# EUROPEAN PATENT APPLICATION

(11) **EP 0 978 561 A1**
(43) Date of publication of application: **09.02.2000**
(21) Application number: 98114854.7
(22) Date of filing: 07.08.1998
(51) Int. Cl.: C12N 15/11, A61K 31/70, C07H 21/00

(54) **Antisense oligonucleotides for the inhibition of VEGF expression**

(71) Applicant: Hoechst Marion Roussel Deutschland GmbH, 65929 Frankfurt am Main (DE)
(72) Inventor: Uhlmann, Eugen, Dr., 61479 Glashütten (DE); Peyman, Anuschirwan, Dr., 65779 Kelkheim (DE); Bitonti, Alan J., Ringoes, New Jersey 08551 (US); Woessner, Richard D., Dr., Lebanon, New Jersey 08833 (US)

(57) **Abstract**

The present invention relates to an oligonucleotide or a derivative thereof which has a sequence that corresponds to a part of a nucleic acid which encodes VEGF (vascular endothelial growth factor) and which has the ability to inhibit tumor growth in animal tumor models, the invention further relates to the preparation of such oligonucleotide and the use thereof.

An oligonucleotide or a derivative thereof which has the sequence SEQ ID NO. 4 or a part thereof, wherein
SEQ ID NO. 4 is 3'-GTACCTACAGATAGTCGCGTCGATGACGGTAGG-5 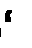 ,
with
the first proviso, that not all internucleoside bridges in the oligonucleotide are phosphodiester internucleoside bridges and not all phosphodiester internucleoside bridges are replaced by phosphorothioate internucleoside bridges and/or the second proviso, that the oligonucleotide contains no modified nucleosides selected from C5-propynyl uridine, C5-propynyl cytidine, C5-hexynyl uridine, C5-hexynyl cytidine, 6-aza uridine and 6-aza cytidine.

## Description

The present invention relates to an oligonucleotide or a derivative thereof which has a sequence that corresponds to a part of a nucleic acid which encodes VEGF (vascular endothelial growth factor) and which has the ability to inhibit tumor growth in animal tumor models, the invention further relates to the preparation of such oligonucleotide and the use thereof

Angiogenesis is defined as the growth of new capillary blood vessels and plays a fundamental role in growth and development. In mature human the ability to initiate an angiogenic response is present in all tissues, but is held under strict control. Angiogenesis is only mobilized in specific situations, such as wound repair and endometrial regulation. The regulation of angiogenesis relies on a fine balance between numerous inhibitory and stimulatory factors. VEGF, also called VPF (vascular permeability factor), is a key regulator of angiogenesis and its mitogenic effect is specific for endothelial cells (Ferrara, Trends Cardiovasc. Med. (1993) 3, 244). VEGF exists in at least four different isoforms that exert similar biological activities and result from alternative splicing. VEGF is expressed in abnormally high levels in human tumors and in diseased tissues characterized by high degree of vascularization or vascular permeability, such as diabetic retinopathy, psoriasis, age-related macular degeneration, rheumatoid arthritis and other inflammatory diseases. Therefore, agents which selectively decrease the VEGF levels may be used to treat malignancies and other angiogenic diseases.

It has been shown that monoclonal antibodies against VEGF can suppress the growth of several tumors in nude mice (Kim et al., Nature (1993) 362, 841). Another possibility for reducing VEGF levels is the use of antisense oligonucleotides, which are optionally modified in order to improve their properties (E. Uhlmann and A. Peyman, Chemical Reviews 90, 543 (1990); S. Agrawal, TIBTECH 1996, 376; EP 0653439 A2). Antisense oligonucleotides are thought to bind to specific sequences of the mRNA resulting in degradation of the mRNA and/or inhibition of protein synthesis.

EP 0769 552 A1 claims antisense oligonucleotides of 8 nucleotides or longer directed against VEGF which can inhibit the expression of VEGF to 30% or less. The oligonucleotides were tested in a cell free system in form of the phosphodiesters, which would not be stable under in vivo conditions. Selected antisense oligonucleotides were also tested in form of the all-phosphorothioates (A085R-S, A087P-S, A227-S, A287-S, A311-S, and A419-S) showing 30 - 46 % inhibition of VEGF expression at 20 µM of all-phosphorothioate oligonucleotide in a A549 cell-based assay.

WO 97/39120 discloses antisense oligonucleotides against VEGF mRNA which reduce cellular VEGF production in treated cells at concentrations of less than about 1 micromolar. In a special embodiment, the antisense oligonucleotide has the sequence (SEQ ID NO. 1) 5'-GCGCTGATAGACATCCATG-3', wherein said oligonucleotide comprises phosphorothioate groups either at all internucleoside bridges or at particular internucleoside positions, and in the latter case in addition at particular positions a modified nucleoside residue selected from C5-propynyl uridine, C5-propynyl cytidine, C5-hexynyl uridine, C5-hexynyl cytidine, 6-aza uridine, or 6-aza cytidine (table 1 in WO 97/39120).

On a meeting of the "American Association for Cancer Research 1998" data relating to the activity of a VEGF antisense oligonucleotide - for which the sequence was not disclosed - was shown " (abstract published in AACR book Vol. 39, p. 95). It was shown, that the oligonucleotide has the ability to inhibit the growth of gliobastoma xenografts in nude mice.

The present invention provides an oligonucleotide or a derivative thereof which has a sequence that corresponds to the sequence SEQ ID NO. 2 or a part thereof wherein SEQ ID NO.2 is 5 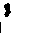 -CATGGATGTCTATCAGCGCAGCTACTGCCATCC-3'. The sequence SEQ ID NO. 2 is a part of a nucleic acid sequence which encodes VEGF. The part of the nucleic acid to which the oligonucleotide (hereinafter "ON') corresponds preferably has a length of 10 to 33 nucleotides, more preferably of 17 to 20 nucleotides, most preferred is a length of 17, 18, 19 or 20 nucleotides. Therefore, an oligonucleotide of the invention preferably has a length of 10 (10 mer) to 33 nucleotides (33 mer), more preferably of 17 to 20 nucleotides, most preferred is a length of 17, 18, 19 or 20 nucleotides (17 mer, 18 mer, 19 mer, 20 mer). In a special embodiment of the invention the oligonucleotide has a length of 19 nucleotides.

The oligonucleotide has a sequence that corresponds to a part of a nucleic acid which encodes VEGF. The phrase "corresponds to" means that the base sequence of the oligonucleotide is complementary to a part of a nucleic acid sequence, that encodes VEGF (e.g. gene, cDNA, mRNA) and therefore allows the oligonucleotide to hybridize to (bind to) that "sense" part of the VEGF encoding nucleic acid (which is preferably a VEGF mRNA). This is why it is called "antisense oligonucleotide". In a preferred embodiment of the invention, the oligonucleotide is an antisense oligonucleotide. In another preferred embodiment of the invention the oligonucleotide is a ribozyme. A ribozyme is a catalytic nucleic acid which cleaves mRNA. Preferred ribozymes are selected from the group of hammerhead ribozymes (Uhlmann and Peyman, 1990).

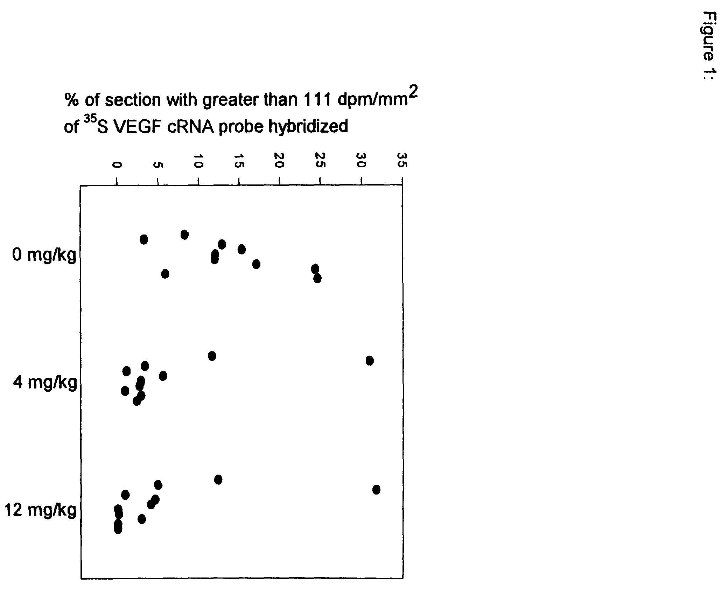

The nucleic acid sequence which encodes VEGF and to which the oligonucleotide corresponds to has the sequence
(SEQ ID NO. 2) 5 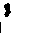 -CATGGATGTCTATCAGCGCAGCTACTGCCATCC-3'.
This sequence is equivalent to nucleotides 185-217 of human VEGF cDNA described in Figure 1B of Leung et al. (Science (1989) 246, 1306). Sequence SEQ ID NO. 2 is equivalent also to nucleotides 185 - 217 of human VEGF mRNA, when the nucleotides are numbered as in Leung et al. (Science (1989) 246, 1306). In a preferred embodiment of the invention, the oligonucleotide is equivalent to nucleotides 185-203 of human VEGF mRNA. A part of the human VEGF cDNA is also given in table 3 (SEQ ID NO. 19); SEQ ID NO. 2 also corresponds to a part of SEQ ID NO. 19.

Therefore, the present invention relates to an oligonucleotide or a derivative thereof which has the sequence SEQ ID NO. 4 or a part thereof, wherein
SEQ ID NO. 4 is 3'-GTACCTACAGATAGTCGCGTCGATGACGGTAGG-5 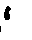 ,
with
the first proviso, that not all internucleoside bridges in the oligonucleotide are phosphodiester internucleoside bridges and not all phosphodiester internucleoside bridges are replaced by phosphorothioste internucleoside bridges and/or the second proviso, that the oligonucleotide contains no modified nucleoside selected from the modified nucleosides C5-propynyl uridine, C5-propynyl cytidine, C5-hexynyl uridine, C5-hexynyl cytidine, 6-aza uridine and 6-aza cytidine. The oligonucleotide corresponds to sequence SEQ ID NO. 2 or a part thereof In a preferred embodiment, the oligonucleotides corresponds to (SEQ ID NO. 3) or a part thereof Preferably an oligonucleotide which corresponds to a part of SEQ ID NO. 3 has a length of 17, 18 or 19 nucleotides.
SEQ ID NO. 3: 5 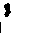 -CATGGATGTCTATCAGCGC-3'
Therefore, the oligonucleotide has for example one of the sequences SEQ ID NO. 4 to SEQ ID NO. 16, wherein
SEQ ID NO. 4 is 3'-GTACCTACAGATAGTCGCGTCGATGACGGTAGG-5 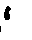 (33mer),
SEQ ID NO. 5 is 3'-CCTACAGATAGTCGCGTCGATGACGG-5 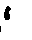 (26 mer),
SEQ ID NO. 6 is 3'-CAGATAGTCGCGTCGATGACGG-5 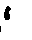 (22 mer),
SEQ ID NO. 7 is 3'-AGTCGCGTCGATGACGG-5 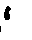 (17 mer),
SEQ ID NO. 8 is 3'-CTACAGATAGTCGCGTCG-5 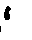 (18 mer),
SEQ ID NO. 9 is 3'-GTACCTACAGATAGTCGCGTCGATGACGG-5 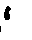 (29 mer),
SEQ ID NO. 10 is 3'-GTACCTACAGATAGTCGCGT-5 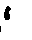 (20 mer),
SEQ ID NO. 11 is 3'-GTACCTACAGATAGTCGCG-5 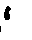 (19 mer)
SEQ ID NO. 12 is 3'-GTACCTACAGATAGTCGC-5 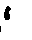 (18 mer),
SEQ ID NO. 13 is 3'-ACCTACAGATAGTCGCG-5 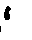 (17 mer),
SEQ ID NO. 14 is 3'-GTACCTACAGATAGTCG-5 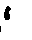 (17 mer),
SEQ ID NO. 15 is 3'-TACCTACAGATAGTCGCG-5 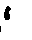 (18 mer), and
SEQ ID NO. 16 is 3'-TACCTACAGATAGTCGC-5 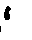 (17 mer).

SEQ ID NO. 4 is the sequence that corresponds to or is complementary to sequence SEQ ID NO. 2, respectively. Sequences SEQ ID NO. 5 to 16 correspond to parts of sequence SEQ ID NO. 2. Sequences SEQ ID NO. 5 to 16 are equivalent to parts of sequence SEQ ID NO. 4. In a preferred embodiment of the invention the oligonucleotide has the sequence SEQ ID NO. 11 (corresponds to a part of a VEGF encoding sequence which has the sequence SEQ ID NO. 3).

The invention also relates to derivatives of the oligonucleotides, for example their salts, in particular their physiologically tolerated salts. Salts and physiologically tolerated salts are e. g. described in Remingtons Pharmaceuticals Science (1985) Mack Publishing Company, Easton, PA (page 1418). Derivatives also relate to modified oligonucleotides which have one or more modifications (e.g. at particular nucleoside positions and/or at particular internucleoside bridges, oligonucleotide analogues (e.g. Polyamide-Nucleic Acids (PNAs), Phosphomonoester nucleic acids (PHONAs = PMENAs), oligonucleotide chimeras (e.g. consisting of a DNA- and a PNA-part or consisting of a DNA- and a PHONA-part)).

A preferred subject of the invention relates to an oligonucleotide which has a sequence that corresponds to SEQ ID NO. 2 or a part thereof (a sequence that is designated from SEQ ID NO. 4 or a part thereof respectively) and which is modified. Most preferably an oligonucleotide is modified in order to improve its properties, e.g. in order to increase its resistance to nucleases or to make it resistant against nucleases, respectively to improve its binding affinity to a complementary nucleic acid) or in order to increase the cellular uptake.

Therefore, the present invention preferably relates to an oligonucleotide which has a particular sequence as outlined above and which has in addition one or more chemical modifications in comparison to a "natural" DNA, which is composed of the "natural" nucleosides deoxyadenosine (adenine + β-D-2*'*-deoxyribose), deoxyguanosine (guanine + β-D-2*'*-deoxyribose), deoxycytidine (cytosine + β-D-2*'*-deoxyribose) and thymidine (thymine + β-D-2*'*-deoxyribose ) linked via phosphodiester internucleoside bridges. The oligonucleotide can have one or more modifications of the same type and/or modifications of a different type; each type of modification can independently be selected from the types of modifications known to be used for modifying oligonucleotides.

Examples of chemical modifications are known to the skilled person and are described, for example, in E. Uhlmann and A. Peyman, Chemical Reviews 90 (1990) 543 and "Protocols for Oligonucleotides and Analogs" Synthesis and Properties & Synthesis and Analytical Techniques, S. Agrawal, Ed, Humana Press, New Jersey, USA 1993 and S.T. Crooke, F. Bennet, Ann. Rev. Pharmacol. Toxicol. 36 (1996) 107-129; J. Hunziker and C. Leuman (1995) Mod. Synt. Methods, 7, 331-417.

For example, in comparison to natural DNA a phosphodiester internucleoside bridge, a β-D-2*'*-deoxyribose and/or a natural nucleoside base (adenine, guanine, cytosine, thymine) can be modified or replaced. An oligonucleotide according to the invention can have one or more modifications, wherein each modification is located at the a particular phosphodiester internucleoside bridge and/or at a particular β-D-2*'*-deoxyribose unit and/or at a particular natural nucleoside base position in comparison to an oligonucleotide of the same sequence which is composed of natural DNA.

For example, the invention relates to an oligonucleotide which comprises one or more modifications and wherein each modification is independently selected from
a) the replacement of a phosphodiester internucleoside bridge located at the 3*'*-and/or the 5*'*- end of a nucleoside by a modified internucleoside bridge,
b) the replacement of phosphodiester bridge located at the 3*'*- and/or the 5*'*-end of a nucleoside by a dephospho bridge,
c) the replacement of a sugar phosphate unit from the sugar phosphate backbone by another unit,
d) the replacement of a β-D-2*'*-deoxyribose unit by a modified sugar unit,
e) the replacement of a natural nucleoside base by a modified nucleoside base,
f) the conjugation to a molecule which influences the properties of the oligonucleotide,
g) the conjugation to a 2 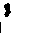 5 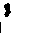 -linked oligoadenylate or a derivative thereof, optionally via an appropriate linker, and
h) the introduction of a 3*'*-3*'* and/or a 5*'*-5*'* inversion at the 3*'* and/or the 5*'* end of the oligonucleotide.

More detailed examples for the chemical modification of an oligonucleotide are
a) the replacement of a phosphodiester internucleoside bridge located at the 3*'*-and/or the 5*'*- end of a nucleoside by a modified internucleoside bridge, wherein the modified internucleoside bridge is for example selected from phosphorothioate, phosphorodithioate, NR¹R¹ 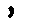 -phosphoramidate, boranophosphate, phosphate-(C₁-C₂₁)-O-alkyl ester, phosphate-[(C₆-C₁₂)aryl-((C₁-C₂₁) -O-alkyl]ester, (C₁-C₈)alkyl-phosphonate and/or (C₆-C₁₂)-arylphosphonate bridges, (C₇-C₁₂)-α-hydroxymethyl-aryl (e.g. disclosed in WO 95/01363), wherein (C₆-C₁₂)aryl, (C₆-C₂₀)aryl and (C₆-C₁₄)aryl are optionally substituted by halogene, alkyl, alkoxy, nitro, cyano, and where R¹ and R¹ 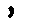 are, independently of each other, hydrogen, (C₁-C₁₈)-alkyl, (C₆-C₂₀)-aryl, (C₆-C₁₄)-aryl-(C₁-C₈)-alkyl, preferably hydrogen, (C₁-C₈)-alkyl and/or methoxyethyl, preferably hydrogen in particular, (C₁-C₄)-alkyl and/or methoxyethyl, or
   R¹ and R¹ 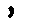 form, together with the nitrogen atom carrying them, a 5-6-membered heterocyclic ring which can additionally contain a further heteroatom from the group O, S and N,
b) the replacement of phosphodiester bridge located at the 3*'*- and/or the 5*'*-end of a nucleoside by a dephospho bridge (dephospho bridges are described, for example, in Uhlmann, E. and Peyman, A. in "Methods in Molecular Biology", Vol. 20, "Protocols for Oligonucleotides and Analogs", S. Agrawal, Ed., Humana Press, Totowa 1993, Chapter 16, 355ff), wherein the dephospho bridges is for example selected from the dephospho bridges formacetal, 3 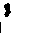 -thioformacetal, methylhydroxylamine, oxime, methylenedimethyl-hydrazo, dimethylenesulfone and/or silyl groups;
c) the replacement of a sugar phosphate unit (β-D-2'-deoxyribose and phosphodiester internucleoside bridge together form a sugar phosphate unit) from the sugar phosphate backbone (sugar phosphate backbone is composed of sugar phosphate units) by another unit, wherein the other unit is for example suitable to built up a
   - "morpholino-derivative" oligomer (as described, for example, in E.P. Stirchak et al., Nucleic Acids Res. 17 (1989) 6129), that is e.g. the replacement by a morpholino-derivative unit;
   - polyamide nucleic acid ("PNA") (as described for example, in P.E. Nielsen et al., Bioconj. Chem. 5 (1994) 3 and in EP 0672677 A2), that is e.g. the replacement by a PNA backbone unit, e.g. by 2-aminoethylglycine;
   - phosphomonoacidic ester nucleic acid ("PHONA") (as described for example, in Peyman et al., Angew. Chem. Int. Ed. Engl. 35 (1996) 2632-2638 and in EP 0739895 A2), that is e.g. the replacement by a PHONA backbone unit;
d) the replacement of a β-D-2*'*-deoxyribose unit by a modified sugar unit, wherein the modified sugar unit is for example selected from β-D-ribose, α-D-2 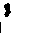 -deoxyribose, L-2 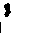 -deoxyribose, 2 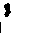 -F-2 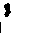 -deoxyribose, 2 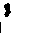 -O-(C₁-C₆)alkyl-ribose, preferably 2'-O-(C₁-C₆)alkyl-ribose is 2'-O-methylribose, 2 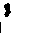 -O-(C₂-C₆)alkenyl-ribose, 2 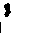 -[O-(C₁-C₆)alkyl-O-(C₁-C₆)alkyl]-ribose, 2 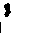 -NH₂-2 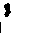 -deoxyribose, β-D-xylo-furanose, α-arabinofuranose, 2,4-dideoxy-β-D-erythro-hexo-pyranose, and carbocyclic (described, for example, in Froehler, J. Am. Chem. Soc. 114 (1992) 8320) and/or open-chain sugar analogs (described, for example, in Vandendriessche et al., Tetrahedron 49 (1993) 7223) and/or bicyclosugar analogs (described, for example, in M. Tarkov et al., Helv. Chim. Acta 76 (1993) 481);
e) the replacement of a natural nucleoside base by a modified nucleoside base, wherein the modified nucleoside base is for example selected from uracil, hypoxanthine, 5-(hydroxymethyl)uracil, N²-Dimethylguanosine, 5-(hydroxymethyl)uracil, 5-aminouracil, pseudouracil, dihydrouracil, 5-fluorouracil, 5-fluorocytosine, 5-chlorouracil, 5-chlorocytosine, 5-bromouracil, 5-bromocytosine, 2,4-diaminopurine, 8-azapurine, a substituted 7-deazapurine, preferably a 7-deaza-7-substituted and/or 7-deaza-8-substituted purine or other modifications of a natural nucleoside bases, e.g. modified nucleoside bases described in EP 0 710 667 A2 and EP 0 680 969 A2;
f) the conjugation to a molecule which influences the properties of the oligonucleotide, wherein the conjugation of the oligonucleotide to one or more molecules which (favorably) influence the properties of the oligonucleotide (for example the ability of the oligonucleotide to penetrate, the cellmembrane or to enter a cell, the stability against nucleases, the affinity for a VEGF encoding target sequence, the pharmacokinetics of the oligonucleotide, the ability of an antisense oligonucleotide/ribozyme to attack the VEGF encoding target sequence, e.g. the ability to bind to and/or to crosslink, when the oligonucleotide or the molecule conjugated to the oligonucleotide respectively hybridizes with the VEGF encoding target sequence), wherein examples for molecules that can be conjugated to an oligonucleotide are polylysine, intercalating agents such as pyrene, acridine, phenazine or phenanthridine, fluorescent agents such as fluorescein, crosslinking agents such as psoralen or azidoproflavin, lipophilic molecules such as (C₁₂-C₂₀)-alkyl, lipids such as 1,2-dihexadecyl-rac-glycerol, steroids such as cholesterol or testosterone, vitamins such as vitamin E, poly- or oligoethylene glycol preferably linked to the oligonucleotide via a phosphate group (e.g. triethylenglycolphosphate), (C₁₂-C₁₈)-alkyl phosphate diesters and/or O-CH₂-CH(OH)-O-(C₁₂-C₁₈)-alkyl. These molecules can be conjugated at the 5 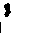 end and/or the 3 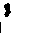 end and/or within the sequence, e.g. to a nucleoside base. Preferred is the conjugation of an oligonucleotide to a a) lipophilic molecule, for example (C₁₂-C₂₀)-alkyl, b) steroid such as cholesterol and/or testosterone, c) poly- and/or oligoethylene glycol, d) with vitamin E, e) intercalating agent such as pyrene, f) (C₁₄-C₁₈)-alkyl phosphate diester and/or g) O-CH₂-CH(OH)-O-(C₁₂-C₁₈)-alkyl. Processes for preparing an oligonucleotide conjugate are known to the skilled person and are described, for example, in Uhlmann, E. & Peyman, A., Chem. Rev. 90 (1990) 543 and/or M. Manoharan in "Antisense Research and Applications", Crooke and Lebleu, Eds., CRC Press, Boca Raton, 1993, Chapter 17, p. 303ff, and/or EP-A 0 552 766;
g) the conjugation to a 2 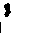 5 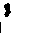 -linked oligoadenylate, preferably via an appropriate linker molecule, wherein the 2*'*5*'*-linked oligoadenylate is for example selected from 2'5'-linked triadenylate, 2'5'-linked tetraadenylate, 2'5'-linked pentaadenylate, 2'5'-linked hexaadenyltat or 2'5'-linked heptaadenylat molecules and derivatives thereof, wherein a 2*'*5*'*-linked oligoadenylate derivative is for example Cordycepin (2 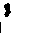 5 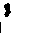 -linked 3 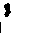 -deoxy adenylate) and wherein an example for an appropriate linker is triethylenglycol and wherein the 5 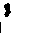 -end of the 2 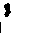 5 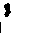 -linked oligoadenylate must bear a phosphate, diphosphate or triphosphate residue in which one or several oxygen atoms can be replaced e.g. by sulfur atoms, wherein the substitution by a phosphate or thiophosphate residue is preferred; and
h) the introduction of a 3*'*-3*'* and/or a 5*'*-5*'* inversion at the 3*'* and/or the 5*'* end of the oligonucleotide, wherein this type of chemical modification is known to the skilled person and is described, for example, in M. Koga et al, J. Org. Chem. 56 (1991) 3757, EP 0 464 638 and EP 0 593 901.

The replacement of a sugar phosphate unit from the sugar phosphate backbone by another unit, which is e.g. a PNA backbone unit or a PHONA backbone unit, is preferably the replacement of a nucleotide by e.g. a PNA unit or a PHONA unit, which already comprise natural nucleoside bases and/or modified nucleoside bases, e.g. one of the modified nucleoside bases (listed under e)) from uracil, hypoxanthme, 5-(hydroxy-methyl)uracil, N²-Dimethylguanosine, 5-(hydroxymethyl)uracil, 5-aminouracil, pseudouracil, dihydrouracil, 5-fluorouracil, 5-fluorocytosine, 5-chlorouracil, 5-chlorocytosine, 5-bromouracil, 5-bromocytosine, 2,4-diamino-purine, 8-azapurine, a substituted 7-deazapurine, preferably 7-deaza-7-substituted and/or 7-deaza-8-substituted purine or other modifications of a natural nucleoside bases, (modified nucleoside bases are e.g. described in EP 0 710 667 A2 and EP 0 680 969 A2).
The modifications described in EP 0 710 667 A2, EP 0 680 969 A2, EP 0 464 638, EP 0 593 901, WO 95/01363, EP 0 672 677 A2, EP 0 739 898 A2 and EP 0 552 766 are hereby incorporated by reference.

An Example for an oligonucleotide which has the sequence SEQ ID NO: 11, modified internucleoside bridges and a 3'3'-inversion at the 3'-end, is
- ON 57:: 5'-G*C*G C*T G A*T*A G A*C*A T*C*C*A*T(3'3')G-3',
wherein
(3'3') is a 3'3'-phosphodiester linkage as described in EP 0 464 638 and "*" is a modified internucleoside bridge.

Examples for oligonucleotides which have the sequence SEQ ID NO: 11 and in which a phosphodiester linkage is replaced by an arylphosphonate bridge are
- ON 58:: 5'-G*C*G C*T G A*T*A G A*C*A T*C*C*A*T(NBP)G-3', and
- ON 59:: 5-G*C*G C*T G A*T*A G A*C*A T*C*C*A*T(NBP)G-3',
wherein
"NBP" is a α-hydroxybenzyl phosphonate linkage, preferably a α-hydroxy-2-nitrobenzyl phosphonate linkage as described in WO 95/01363 and "N" is a 2'-O-alkylribonucleoside, preferably a 2'-O-methylribonucleoside (in this case "T" is 2*'*-O-alkluridine, preferably 2*'*-O-methyluridine).

Examples for oligonucleotides which have the sequence SEQ ID NO: 11 and in which a nucleoside base is replaced and by a modified nucleoside base like described in EP 0 710 667 and EP 0 680 969 are
- ON 60:: 5'-G*C*G C*T G a*T*a g a*C*a T*C*C*A*T*G-3' and
- ON 61:: 5'-G*C*G C*T G A*T*A G a*C*a T*C*C*A*T*G-3',
wherein
a lower case letter "a" is a 8-aza-deoxyadenosine or a optionally substituted 7-deaza-deoxyadenosine and a lower case letter "g" is a 8-aza-deoxyguanosine or a optionally substituted 7-deaza-deoxyguanosine (examples for base modifications as described in EP 0 710 667 A2 and EP 0 680 969 A2) and wherein a "N" is a 2'-O-alkylribonucleoside, preferably a 2'-O-methylribo-nucleoside (in this case "T" is 2*'*-O-alkluridine, preferably 2*'*-O-methyluridine).

In a special embodiment of the invention, at least one or more internucleoside bridges within the oligonucleotide sequence are modified, preferably with phosphorothioate. In an all-phosphorothioate oligonucleotide, all phosphodiester internucleoside bridges are modified by phosphorothioate. Preferably, the invention relates to an oligonucleotide in which not all phosphodiester internucleoside bridges are modified uniformly with phosphorothioate (phosphorothioate internucleoside bridges), especially not, if the oligonucleotide has the sequence SEQ ID NO. 11. Preferably, at least one internucleoside bridge has a different type of modification or is not modified.

In a preferred embodiment of the invention only particular positions within an oligonucleotide sequence are modified (e.g. partially modified oligonucleotide). Partially modified oligonucleotides are also named minimal modified oligonucleotides in some documents. Within the sequence a modification can be located at particular positions (at particular nucleotides, at particular nucleosides, at particular nucleoside bases, at particular internucleoside bridges).

In a particular embodiment of the invention, an oligonucleotide is prepared by only replacing some of the phosphodiester bridges with modified internucleoside bridges, e.g. phosphorothioate bridges. In particular, the invention comprises such oligonucleotides which are only modified to a certain extent.

Oligonucleotides ON 1 and ON 62-73 are examples for the localization of modified internucleoside bridges with respect to sequences SEQ ID NO. 4 to SEQ ID NO: 16:
- ON 62:: 3'-G*T*A*C*C*TAC*AGA*TAGT*C*GC*GT*C*GAT*GAC*GGT*AGG-5 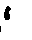 (example for SEQ ID NO. 4),
- ON 63:: 3'-C*C*T*AC*AGAT*AGT*C*GC*GT*C*GAT*GAC*G*G-5 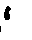 (example for SEQ ID NO. 5),
- ON 64:: 3'-C*A*GA*TAGT*C*G*CGT*C*GAT*GAC*G*G-5 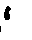 (example for SEQ ID NO. 6),
- ON 65:: 3'-A*G*T*CGC*GT*C*GA*T*GAC*G*G-5 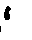 (example for SEQ ID NO. 7),
- ON 66:: 3'-C*T*A*CAGA*TAGT*C*GC*GT*C*G-5 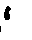 (example for SEQ ID NO. 8),
- ON 67:: 3'-G*T*AC*C*TAC*AGAT*AGT*C*GC*GT*C*GAT*GAC*G*G-5 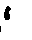 (example for SEQ ID NO. 9),
- ON 68:: 3'-G*T*AC*C*TAC*AGAT*AGT*C*GC*G*T-5 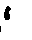 (example for SEQ ID NO. 10),
- ON 1:: 3'-G*T*A*C*C*TA*C*AGA*T*AGT*CG*C*G-5' (example for SEQ ID NO. 11),
- ON 69:: 3'-G*T*A*C*C*TA*C*AGA*T*AGT*C*G*C-5 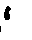 (example for SEQ ID NO. 12),
- ON 70:: 3'-A*C*C*T*AC*AGA*T*AGT*C*G*C*G-5 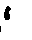 (example for SEQ ID NO. 13),
- ON 71:: 3'-G*T*A*C*C*TA*C*AGA*T*AG*T*C*G-5 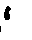 (example for SEQ ID NO. 14),
- ON 72:: 3'-T*A*C*C*TAC*AGA*T*AG*T*CG*C*G-5 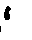 (example for SEQ ID NO. 15) and
- ON 73:: 3'-T*A*C*C*TAC*AGA*T*AG*T*C*G*C-5 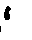 (example for SEQ ID NO. 16),
wherein
" * " shows the localization of the internucleoside bridge modification within the sequence. In a preferred embodiment of the invention the type of modification is the replacement of phosphodiester bridges by phosphorothioate bridges, in this case " * " shows the position of a phosphorothioate internucleoside bridge. A preferred embodiment of the invention relates to ON 1, wherein "*" is a phosphorothioate bridge:

- ON 1:: 3'-G*T*A*C*C*TA*C*AGA*T*AGT*CG*C*G-5',
wherein
"*" is a phosphorothioate bridge.

In a particular embodiment the invention relates to an oligonucleotide, wherein the terminal 1 to 5 nucleotide units at the 5*'* end and/or at the 3*'* end of the oligonucleotide are protected by modifying internucleoside bridges located at the 5*'*and/or the 3*'* end of the corresponding nucleosides. Most preferably the terminal 1 to 5 nucleotide units at the 3*'* end of the oligonucleotide are protected by modifying internucleoside bridges located at the 5*'*and/or the 3*'* end of the corresponding nucleosides. Optionally, the terminal 1 to 5 nucleotide units at the 5*'* end of the oligonucleotide are in addition protected by modifying internucleoside bridges located at the 5*'*and/or the 3*'* end of the corresponding nucleosides. Optionally, the oligonucleotide may comprise additional modifications at other positions. An example for an oligonucleotide which has the sequence SEQ ID NO. 11 and such pattern of modification is
- ON 2:: 5'- G*C G*C*T*G*A*T*A G*A*C*A*T*C*C*A*T*G -3*'*
wherein
"*" indicates the localization of the internucleoside bridge modifications, preferably "*" is a phosphorothioate bridge.

Further, the invention relates to an oligonucleotide, wherein at least one internal pyrimidine nucleoside and/or an internucleoside bridge located at the 5*'*end of this pyrimidine nucleoside and/or located at the 3*'*end of this pyrimidine nucleoside is modified.

In a preferred embodiment of the invention the terminal 1 to 5 nucleotide units at the 5*'* end and/or at the 3*'* end of the oligonucleotide are protected by modifying internucleoside bridges located at the 5*'*and/or the 3*'* end of the corresponding nucleosides and wherein in addition at least one internal pyrimidine nucleoside and/or an internucleoside bridge located at the 5*'*end of this pyrimidine nucleoside and/or located at the 3*'*end of this pyrimidine nucleoside is modified.

The principle of partially modified oligonucleotides is described in A. Peyman, E. Uhlmann, Biol. Chem. Hoppe-Seyler, 377 (1996) 67-70 and in EP 0 653 439. This document is hereby incorporated by reference. In this case, 1-5 terminal nucleotide units at the 5 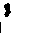 end/or and at the 3 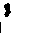 end are protected, e.g. the phosphodiester internucleoside bridges located at the 3*'*and/or the 5*'*end of the corresponding nucleosides are for example replaced by phosphorothioate internucleoside bridges. In addition, preferably at least one internal pyrimidine nucleoside (or nucleotide respectively) position is modified; preferably the 3*'* and/or the 5*'* internucleoside bridge of a pyrimidine nucleoside is modified, for example by phosphorothioate. Partially modified oligonucleotides exhibit particularly advantageous properties; for example they exhibit a particularly high degree of nuclease stability in association with minimal modification. They also have a significantly reduced propensity for non-antisense effects which are often associated with the use of all-phosphorothioate oligonucleotides (Stein and Krieg (1994) Antisense Res. Dev. 4, 67). Partially modified oligonucleotides also show a higher binding affinity than all-phosphorothioates.

The invention relates in particular to partially/minimally modified oligonucleotides. Examples for the internucleoside modification pattern of partially modified oligonucleotides which have the sequence SEQ ID NO. 11 are:
- ON 3:: 5'-G*C*G C*T*G A*T*A G A*C*A T*C*C*A*T*G -3*'*,
- ON 4:: 5'- G*C*G*C*T*G A*T*A G A*C*A T*C*C*A*T*G -3*'*,
- ON 5:: 5'- G*C G C*T*G A*T*A G A*C*A T*C*C*A*T*G -3*'*,
- ON 6:: 5'- G*C G C*T G A*T*A G A*C*A T*C*C*A*T*G -3*'*,
- ON 7:: 5'- G*C*G C*T G A*T*A G A*C*A T*C*C*A*T*G -3*'*,
- ON 8:: 5'- G C*G C*T G A*T A G A*C*A T*C*C A*T*G -3*'*,
- ON 9:: 5'- G*C*G C*T G A*T*A G A*C*A T*C*C A*T*G -3*'*,
- ON 10:: 5'- G*C*G C*T G A*T*A G A*C*A*T*C C*A*T*G -3*'*,
- ON 11:: 5'- G*C*G*C*T G A*T*A G A*C*A T*C*C*A*T*G -3*'*,
- ON 12:: 5'- G*C*G*C*T G A*T*A G A*C A*T*C*C*A*T*G -3*'*,
- ON 13:: 5'- G*C*G C*T G A*T*A G A*C*A T*C*C*A*T*G -3*'*,
- ON 14:: 5'- G*C*G*C*T G A*T*A G A*C*A*T*C*C*A*T*G -3*'* and
- ON 15:: 5'- G*C G*C*T*G*A*T*A*G*A*C*A*T*C*C*A*T*G -3*'*,
wherein
" * " denotes the localization of the internucleoside bridge modification; preferably " * " is a phosphorothioate bridge.

Similar patterns of internucleoside bridge modifications are also possible for other oligonucleotides according to the invention, which have a different sequence, e.g. one of the sequences SEQ ID NO. 4 to SEQ ID NO. 16.

According to the invention, the oligonucleotides can have in addition to one type of modification, also other types of modification. For example, a partially modified oligonucleotide which has modifications at particular internucleoside bridges, may also have an additional modification, e.g. modification of a β-D-2*'*-deoxyribose or modification of a natural nucleoside base.

Therefore, another example for a special embodiment of the invention relates to a partially modified oligonucleotide which has a modification of a nucleoside, e.g. a modification of a nucleoside base and/or a modification of a β-D-2*'*-deoxyribose unit. Preferably a β-D-2*'*-deoxyribose is replaced by 2 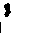 -O-(C₁-C₆)alkylribose, most preferred is the replacement by 2'-O-methylribose (replacement of β-D-2*'*-deoxyribonucleoside by 2*'*-O-methylribonucleoside). Examples of such oligonucleotides which have e.g. sequence SEQ ID NO. 11 can display the following patterns of nucleoside modifications
- ON 16:: 5'- G C G C T G A T A G A CA T C C A T G -3',
- ON 17:: 5'- G C G C T G A T A G A CA T C C A T G -3',
- ON 18:: 5'- G C G C T G A T A G A CA T C C A T G -3',
- ON 19:: 5'- G C G C T G A T A G A CA T C C A T G -3',
- ON 20:: 5'- G C G C T G A T A G A CA T C C A T G -3',
- ON 21:: 5'- G C G C T G A T A G A CA T C C A T G -3',
- ON 22:: 5'- G C G C T G A T A G A CA T C C A T G -3',
- ON 23:: 5'- G C G C T G A T A G A CA T C C A T G -3',
- ON 24:: 5'- G C G C T G A T A G A CA T C C A T G -3',
- ON 25:: 5'- G C G C T G A T A G A CA T C C A T G -3',
- ON 26:: 5'- G C G C T G A T A G A CA T C C A T G -3', and
- ON 27:: 5'- G C G C T G A T A G A CA T C C A T G -3',
wherein
" N " indicates the position of a modified nucleoside (e.g. modification of the nucleoside base and/or modification of the β-D-2*'*-deoxyribose, preferably 2*'*-O-alkyribonucleoside, preferably 2*'*-O-methlyribonucleotides (in this case "T" is 2*'*-O-alkluridine, preferably 2*'*-O-methyluridine).

Similar patterns of nucleoside modifications are also possible for other oligonucleotides according to the invention, which have a different sequence, e.g. one of the sequences SEQ ID NO. 4 to SEQ ID NO. 16.

In another preferred embodiment of the invention the oligonucleotide comprises modified internucleoside bridges at particular positions (preferably a phosphorothioate bridge) and in addition modification of a nucleoside at particular positions, preferably the replacement of β-D-2*'*-deoxyribose by 2'-O-(C₁-C₆) alkylribose. In a preferred embodiment of the invention, the internucleoside modification is the replacement of a phosphodiester bridge by a phosphorothioate bridge and the modification of the β-D-2*'*-deoxyribose is the replacement by 2*'*-O-methylribose; in this case, the oligonucleotide is a chimeric oligonucleotide, which is composed of modified and unmodified DNA and RNA parts - which comprise phosphorodiester and phosphorothioate internucleoside bridges and the nucleosides 2 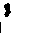 -O-methyl-ribonucleoside and β-D-2*'*-deoxyribonucleoside. Examples for such oligonucleotides, which have the sequence SEQ ID NO. 11 and modifications at particular internucleoside bridges and in addition at particular nucleoside positions are (examples for patterns of modifications):
- ON28:: 5'- G*C*G C*T G A*T*A G A*C*A T*C*C*A*T*G -3',
- ON29:: 5'- G*C*G C*T G A*T*A G A*C*A T*C*C*A*T*G -3',
- ON30:: 5'- G*C*G C*T G A*T*A G A*C*A T*C*C*A*T*G-3',
- ON31:: 5'- G*C*G C*T G A*T*A G A*C*A T*C*C*A*T*G-3',
- ON32:: 5'- G*C^{*}G C^{*}T G A*T*A G A*C*A T*C*C*A*T*G -3',
- ON33:: 5'- G*C*G C*T G A*T*A G A*C*A T*C*C*A*T*G -3',
- ON34:: 5'- G*C*G C^{*}T G A*T*A G A*C*A T*C*C*A*T*G -3',
- ON35:: 5'- G*C*G C*T G A*T*A G A*C*A T*C*C*A*T*G -3',
- ON36:: 5'- G*C*G C*T G A*T*A G A*C*A T*C*C*A*T*G -3',
- ON37:: 5'- G*C*G C T G A*T*A G A*C*A T*C*C*A*T*G -3' and
- ON38:: 5'- G*C*G C*T G A*T*A G A*C*A*T*C*C*A*T*G -3',
wherein
" * " shows the position of the internucleoside bridge modification and "N" is a modified nucleoside (e.g. modification of the nucleoside base and or modification of the β-D-2*'*-deoxyribose);
preferably, "*" is a phosphorothioate bridge and
"N" indicates the position of a 2'-O-alkylribonucleoside, preferably a 2'-O-methylribonucleoside (in this case "T" is 2*'*-O-alkluridine, preferably 2*'*-O-methyluridine).

Comparable modification patterns are also possible for other oligonucleotides according to the invention, which have a different sequence, e.g. one of the sequences SEQ ID NO. 4 to SEQ ID NO. 16.

The invention also relates to derivatives, in which the last nucleotide at the 3'-end is a 2'-deoxynucleotide, e.g.
- ON 39:: 5'-G*C*G C*T G A*T*A G A*C*A T*C*C*A*T*G -3'
wherein
"*" and "N" has the same meaning as above.

In another preferred embodiment of the invention the oligonucleotide comprises a modification of the sugar phosphate backbone, preferably by PNA units. Examples of such PNA-DNA chimeras, which have sequence SEQ ID NO. 11 display the following patterns of modifications (for their general design see EP 0 672 677):
- ON 40:: 5'-G C G C T G A T A g a ca t c c a t g -3' (Pattern: DNA-PNA),
- ON 41:: 5'- G C G C T G a t a g a ca t c c a t g₋-3' (Pattern: DNA-PNA) and
- ON 42:: 5'- g c g c t g A T A G A CA t c c a t g -3' (Pattern: PNA-DNA-PNA),
wherein the lower case letters indicate PNA units.

Also other patterns of modifications are possible e.g. DNA-PNA-DNA, PNA-DNA. Comparable patterns of modification are also possible for PHONA/DNA chimeras. These modification patterns can be combined with any other type of modification and of course, similar patterns of modification are also possible for other oligonucleotides according to the invention, which might have a different sequence, e.g. one of the sequences SEQ ID NO. 4 or SEQ ID NO. 16.

In another preferred embodiment of the invention the oligonucleotide comprises the combination of replacement by PNA units with the replacement of deoxyribonucleosides, e.g. by 2'-O-alkyl-ribonucleosides. Examples for such oligonucleotides which all have sequence SEQ ID NO. 11 are
- ON 43:: 5'- G C G C T G A T A G A Ca t c c a t g₋-3',
- ON 44:: 5'- G C G C T G A T A G A CA T C C A T G -3',
- ON 45:: 5'- G C G C T G A T A G A CA T C C A t g -3',
- ON 46:: 5'- G C G C T G A T A G A CA T C C A t g -3' or
- ON 47:: 5'- G C G C T G A T A G A CA t c c a t g₋-3'
wherein
" N " indicates the position of a modified nucleoside, e.g. of a 2'-O-(C₁-C₆)alkylribonucleoside, preferably a 2'-O-methylribonucleoside (in this case "T" is 2*'*-O-alkluridine, preferably 2*'*-O-methyluridine) and wherein the lower case letters indicate PNA units.

Of course, such oligonucleotides may have in their DNA part also modifications of internucleoside bridges. For example, the above oligonucleotides ON40, ON41, ON42, ON43, ON 44, ON45, ON46 and ON47 may have the internucleoside bridge modifications shown in Oligonucleotides ON1, ON2, ON3, ON4, ON5, ON6, ON7, ON8, ON9, ON10, ON11, ON12, ON13, ON14 and ON15 (Combination of modification pattern of the oligonucleotides ON40-ON47 with one of the modification pattern of ON1-ON15). Similar modification patterns are possible for oligonucleotides according to the invention, which have a different sequence, e.g. on the sequences SEQ ID NO. 4 to SEQ ID NO.16.

A further preferred embodiment of the invention provides an oligonucleotide which has one or more (C₁₂-C₁₈)-alkyl residues, preferably a C₁₆-alkyl residue at its 3 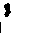 and/or its 5 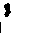 end. A (C₁₂-C₁₈)-alkyl residue can e.g. be bound as a phosphodiester as described in EP 0 552 766 A2 or as a 3 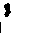 -phosphodiester of O-CH₂-CH(OH)-O-(C₁₂-C₁₈)-alkyl. A preferred embodiment is an oligonucleotide that has a C₁₆ -alkyl residue bound to its 3 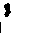 - and/or 5 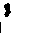 -end. EP 0 552 766 A2 is hereby incorporated by reference.
Examples for such oligonucleotides are ON48 and ON49 (both having the sequence SEQ ID NO. 11 and internucleoside modifications at particular positions, like e.g. in ON1 and in addition a C₁₆ -alkyl linked either to its 5*'*end or to its 3*'*end) (such oligonucleotide might also have any other sequence and pattern of modification):
- ON 48:: 5'- G*C*G C*T G A*T*A G A*C*A T*C*C*A*T*G -C₁₆ -3'
- ON 49:: 5'- C₁₆ -G*C*G C*T G A*T*A G A*C*A T*C*C*A*T*G -3'

The invention also relates to derivatives, in which the 3'-terminus is modified as a triethylenglycol (TEG) phosphodiester, an example for such an oligonucleotide, which has the sequence SEQ ID NO. 11 and the pattern of modification of ON39; is
- ON50:: 5'-G*C*G C*T G A*T*A G A*C*A T*C*C*A*T*Gp-TEG-3'.

In another specific embodiment of the invention the oligonucleotide is connected via a linker to a 2 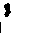 5 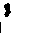 -linked oligoadenylate-5 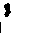 -(thio)phosphate. The linker can e.g. be an oligo-ethylenglycol-phosphate, preferably triethylenglycol-phosphate, tetra-ethylenglycol-phosphate or hexa-ethylenglycol-phosphate residue. The 2 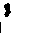 5 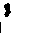 -linked oligoadenylate is preferably attached via its 2 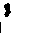 -end as a tetra- or as a pentaadenylate whose 5 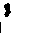 -hydroxy function is substituted by a phosphate or thiophosphate residue. The 2 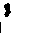 5 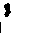 -oligoadenylate is known to induce RNase L to cleave the target mRNA (Torrence et al., Proc. Natl. Acad. Sci. U.S.A. (1993) 90, 1300). The 2 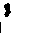 5 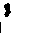 -oligoadenylate serves the purpose to activate ribonuclease L (RNase L) which then degrades the VEGF mRNA. Instead of a 2 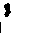 5 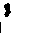 -linked adenylate, a 2 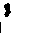 5 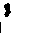 -linked 3 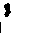 -deoxy adenylate, derived from the nucleoside analog cordycepin, can also be introduced. In this case, the oligonucleotide part, which is complementary to the target nucleic acid is preferably modified at particular positions by 2 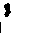 -O-(C₁-C₆)alkylribonucleoside (preferably 2'-O-methylribonucleoside) or by PNA. Examples for such oligonucleotides, which can have e.g. the sequence SEQ ID NO. 11 are ON51 and ON52 (such oligonucleotide might also have any other sequence of an oligonucleotide according to the invention):
- ON51:: 5'-p*(2'5'-Co Co Co Co)(teg) G C G C T G A T A G A C A T C C A T G -3*'*
- ON52:: 5'-p*-(2'5'-rA rA rA rA)(teg) G C G C T G A T A G A C A T C C A T G -3*'*
wherein
"teg" is triethyleneglycol (linker),
"rA" is ribo-A (2 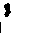 5 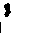 -linked adenylate),
"Co" is 3'-deoxy-A (Cordycepin) (2 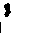 5 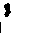 -linked 3 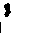 -deoxy adenylate) and
"p*" is 5'-thiophosphate.

In addition, such an oligonucleotide might have further modifications, for example the oligonucleotide might have the pattern of modification of ON 38 (such oligonucleotide might have also any other pattern of modification). Examples are
- ON53:: 5'-p*(2'5'-Co Co Co Co)(teg) G*C*G C***T** G A*T*A G A*C*A T*C*C*A*T*G -3*',* and
- ON54:: 5'-p*-(2'5'-rA*rA*rA*rA)*(teg) G*C*G C*T G A*T*A G A*C*A T*C*C*A*T*G -3*',*
wherein
"teg^{"} is triethyleneglycol (linker),
"N " is a modified nucleoside, preferably a 2'-O-methylribonucleoside (in this case "T" is 2*'*-O-alkluridine, preferably 2*'*-O-methyluridine),
"rA" is ribo-A (2 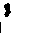 5 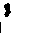 -linked adenylate),
"Co" is 3'-deoxy-A (Cordycepin) (2 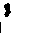 5 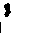 -linked 3 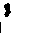 -deoxy adenylate),
"p*" is 5'-thiophosphate, and
"*" is a modified internucleoside bridge, preferably a phosphorothioate internucleoside bridge.

In a preferred embodiment of the invention an oligonucleotide according to the invention can inhibit the expression of the target protein (which is VEGF) or the target sequence (a nucleic acid which encodes VEGF, preferably VEGF mRNA) respectively. Preferably an oligonucleotide according to the invention specifically inhibits the expression of VEGF. This results in a reduction in the VEGF protein level in comparison to untreated expression. The specificity can for example be demonstrated by determining the effect of an oligonucleotide according to the invention upon VEGF expression in comparison to the effect of the same oligonucleotide upon beta actin expression on the mRNA and/or the protein level: upon treatment with an oligonucleotide according to the invention only the VEGF mRNA and/or protein levels were reduced, while e.g. beta actin (a house-keeping protein) mRNA and/or protein levels remained unchanged. In particular, the effect of an oligonucleotide can be demonstrated by determining the VEGF mRNA and/or the VEGF protein amount (e.g. in comparison to a parallel experiment without the oligonucleotide). For example, the inhibitory effect of the oligonucleotide can be determined in vitro by treating cell cultures with the oligonucleotide. Then, for example the mRNA level can be determined in cell lysate preparations, for example as described in example 6. The VEGF protein level (e.g. absolute amount of VEGF protein in gram or e.g. relative in comparison to an untreated cell in percent) can for example be determined from the supernatant (e.g. the culture medium) (the amount of secreted VEGF) and/or membrane preparations (the amount of membrane-bound VEGF) and/or cell lysates. The amount of secreted VEGF protein can for example be determined by ELISA, e.g. as described in example 5.

In a particular embodiment of the invention, an oligonucleotide can inhibit the expression of VEGF mRNA and/or reduce the VEGF protein level respectively, e.g. in a cell culture with an IC₅₀ of about 1µM and/or 500 nM, 200 nM, 100 nM or less.

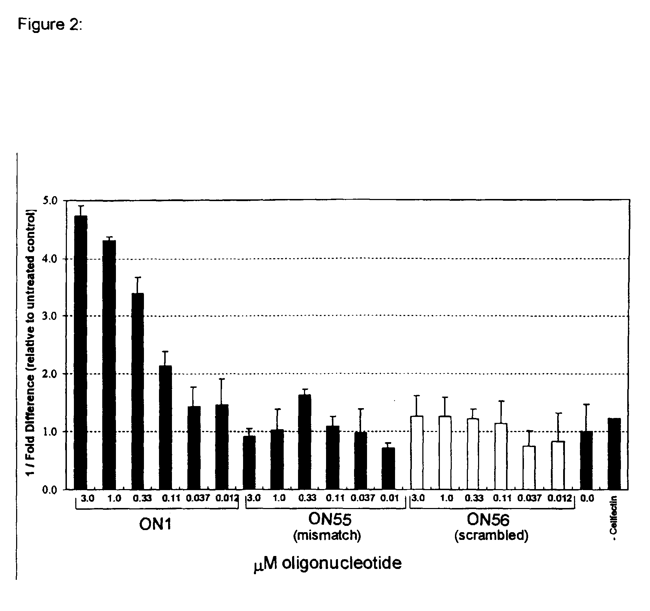

In another particular embodiment of the invention, the inhibition is specific for an oligonucleotide according to the invention; in these cases only the oligonucleotide which has a sequence according to the invention reduces the VEGF protein and/or VEGF mRNA level. In comparison to these specific oligonucleotides, these levels do not change to the same extend, if they change significantly at all, when an oligonucleotide with a mismatch or a scrambled sequence is used. Such oligonucleotides are used as control oligonucleotides, like oligonucleotides ON 55 and ON 56. ON 55 is a mismatch control with respect to SEQ ID NO. 11; it has the sequence SEQ ID NO. 17 and phosphorothioate modifications at particular positions (" * ") ON 56 is a scrambled control with respect to SEQ ID NO. 11; it has the sequence SEQ ID NO. 18 and phosphorothioate modifications at particular positions (" * "). These two oligonucleotides are used e.g. in comparative experiments with ON 1 (table 1 and Figure 2). The control oligonucleotides do not inhibit the expression of VEGF mRNA in cell culture at a concentration of 1 µM and lower.
SEQ ID NO. 17 with phosphorothioate modifications at particular positions (ON55):
   - ON55:: 5'- G*C*G A*C G A*T*A G A*T*C T*C*C*A*T*G -3'
SEQ ID NO. 18 with phosphorothioate modifications at particular positions (ON56):
   - ON56:: 5'- C*G*A A*G C*A*C*T G*T A*C G*C*A T*T*G -3'

In addition, the partial phosphorothioated oligonucleotide ON 1 which has natural nucleoside bases shows a different pharmacological profile as the partial phosphorothioate oligonucleotide that has in addition C5-propynyl uracil and C5-propynyl cytosine base replacements. Such C5-propynyl uracil and C5-propynyl cytosine base analog are e.g. described in WO97/39120:
5'- G*C*G C*T G A*T*A G A*C*A*T*C C*A*T*G -3'
   - underlined C:: 5-propynyl dC, T: 5-propynyl dU,
   " * " phosphorothioate bridges

Several oligonucleotides with different types of modifications were tested (ON 1, ON 28, ON 29, ON 53 and ON 54). The results in table 1 demonstrate, that oligonucleotides according to the invention (right sequence and different types of modifications) efficiently inhibit VEGF protein synthesis in cell culture relative to control oligonucleotides (ON 55, ON 56). As described in example 4, cells were treated with oligonucleotides ON1, ON28, ON29, ON53, ON54, ON55 and ON56 and then the supernatant was assayed for the amount of secreted VEGF protein. In this cell based assay the oligonucleotide ON54 showed the lowest IC₅₀ value, which was about 230 nM. Also ON1 and ON29 showed very good IC₅₀ values, which were about 300 nM and for ON29 and ON53 IC₅₀ values of 500nM and 1500nM respectively were determined. All modified oligonucleotides according to the invention which were tested, showed much better results than control oligonucleotides ON55 and ON56 (IC₅₀ > 3 µM). For ON1 in addition the effect on the mRNA level was tested. For ON1 IC₅₀ value of about 100 nM was determined for reducing the mRNA concentration about 50 %. In comparison, ON55 and ON56 which have a different sequence, but the same type modification and a similar pattern of modification, no effect on the mRNA level was found. Also all three oligonucleotides (ON1, ON55 and ON 56) had no effect on the β-actin mRNA level. Therefore, the effect of ON1 is specific for VEGF mRNA and the effect on VEGF mRNA is specific for a particular oligonucleotide sequence. Furthermore, this is a hint to mechanism by which ON1 acts. Since the VEGF mRNA level is specifically reduced upon treatment with ON1, binding of ON1 to VEGF mRNA most probably acivates RNase H or binding provides a substrate for RNase H respectively. For other types of oligonucleotides with different types of modifications, the mechanism of action which at the end leads to a decrease in VEGF protein level might be different.

An oligonucleotide according to the invention, when administered to a vertebrate, inhibits the expression of VEGF. Therefore, an oligonucleotide according to the invention has the ability to inhibit tumor growth in vertebrates, in particular in human and mice to a certain extent. The specificity of inhibition of VEGF expression can e.g. be determined by measuring the VEGF mRNA and/or VEGF protein levels in tumors of treated individuals relative to untreated individuals. The inhibition of tumor growth can e.g. be determined by measuring the reduction of tumor volume of treated animals versus non-treated animals.

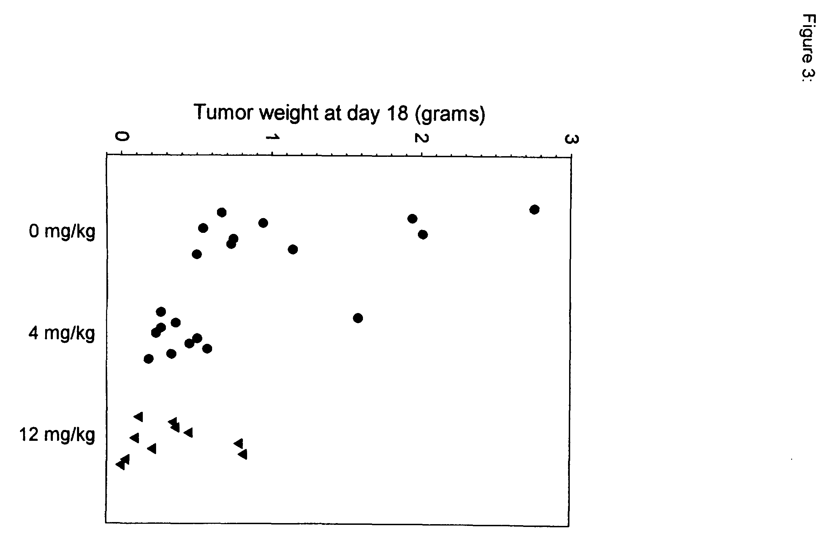

The oligonucleotide can be used for treating a vertebrate at a concentration of 20 mg/kg body weight, preferably at a concentration of 12 mg/kg body weight or less, most preferably at a concentration of about 4 mg/kg body weight or less (table 2, Figure 3). In a special embodiment of the invention, the oligonucleotide or a pharmaceutical composition thereof has the ability to reduce tumor volume at least 30 %, preferably more than 50 % in comparison to untreated individuals after 17 days of administering an oligonucleotide according to the invention to the individual at a concentration of 4 or 12 mg/kg body weight. In order to determine the ability of the oligonucelotide to inhibit a human tumor grown in a vertebrate, a study with a non-human vertebrate which is preferably a mice can be performed. In such a study, a tumor xenograft can e.g. be grown for 1 to 4 days in a non-human vertebrate previous to the administration of the oligonucleotide. The oligonucleotide or a pharmaceutical composition thereof can be administered to such non-human vertebrate.
Preferably, the effect of an oligonucleotide on tumor volume is determined by using a nude mice, with an U87-MG xenograft, made by implanting 2 x 10⁶ human U87-MG cells on day O. U87-MG xenografts grow rapidly in nude mice and the growth of U57-MG xenografts is VEGF dependent.

The invention also relates to a method for the preparation of an oligonucleotide according to the invention. A method for preparation comprises the chemical synthesis of the oligonucleotide. Preferably the chemical synthesis is performed by a standard method known to be used for the synthesis of oligonucleotides, , e.g. the phoshoramidite method according to Caruthers (1983) Tetrahedron Letters 24, 245, the H-phosphonate methode (Todd et at. (1957) J. Chem. Soc. 3291 or the phosphotriester methode (Sonveaux (1986) Bioorg. Chem. 14,274; Gait, M.J. "Oilgonucleotide Synthesis, A practical Approach", IRL Press, Oxford,1984) or improved or varied methods derived from these standard methods. An oligonucleotide according to the invention can for example be prepared as described in examples 1, 2 and 3. Preferably an oligonucleotide according to the invention is synthesized on a solid support by condensing suitably protected monomers (e.g. nucleosides) in order to form internucleoside bridges between these monomers.

The invention relates e.g. to a method for preparing an oligonucleotide or a derivative thereof, where a nucleotide unit with a 3*'*- or a 2*'*-terminal phosphorus (V) group and a free 5*'*-hydroxyl or mercapto grouping is reacted with a further nucleotide unit with a phosphorus (III) or a phosphorus (V) grouping in the 3*'*position, or its activated derivatives and wherein optionally protective groups are used, which can be temporarily introduced in the oligonucleotide in order to protect other functions and which are removed after synthesis, than the oligonucleotide which has been cleaved from the solid support can optionally be converted into a physiologically tolerated salt.
In order to introduce the modifications, standard methods are varied to a certain extend. Those variations are known to a person of skill in the art and are e.g. described in Agrawal S. "Protocols for oligonucleotides and analogs" (1993, Human Press Inc., Totowa, New Jersey, USA). The preparation of modified oligonucleotides is also described in EP 0 710 667, EP 0 680 969, EP 0 464 638, EP 0 593 901, WO 95/01363, EP 0 672 677, EP 0 739 898 and EP 0 552 766. The methods of preparing modified oligonucleotides described in the above documents are hereby incorporated by reference.

The invention further relates to a method of inhibiting the expression of VEGF and/or modulating the expression of a VEGF encoding nucleic acid, wherein an oligonucleotide according to the invention is brought into contact with a VEGF encoding nucleic acid (e.g. mRNA, cDNA) and the oligonucleotide is hybridized to (bind to) this VEGF encoding nucleic acid.

Therefore, the invention also relates to a method, wherein the oligonucleotide is brought into contact with a VEGF encoding nucleic acids (e.g. mRNA; cDNA), for example by introducing the oligonucleotide into a cell by known methods, for example by incubation of cells with said oligonucleotide or a formulation thereof - such formulation may comprise uptake enhancers, such as lipofectin, lipofectamine, cellfectin or polycations (e.g. polylysine).

For example, an oligonucleotide which was incubated previously with cellfectin for e.g. 30 minutes at room temperature is then incubated about 5 hours or less with a cell in order to introduce the oligonucleotide into the cell.

The invention further relates to the use of the oligonucleotide, preferably as antisense oligonucleotide (binding of the oligonucleotide to a VEGF encoding mRNA) or as ribozyme (binding to a VEGF encoding mRNA and cleavage of this mRNA). In another special embodiment of the invention, the oligonucleotide can be used to induce RNAse H cleavage of the VEGF encoding mRNA, thus resulting a reduction in VEGF expression.

The invention relates to the use of the oligonucleotide for modulating and also totally or partially inhibiting the expression of VEGF (e.g. VEGF₁₂₁, VEGF₁₆₅, VEGF₁₈₉, VEGF₂₀₆) and/or splice variants thereof and/or mutants thereof, for example for totally or partially inhibiting translation of VEGF encoding mRNA.

The invention relates to the use of an oligonucleotide for inhibiting, preventing or modulating angiogenesis, neovascularisation, tumor growth and metastasis, in particular in vertebrate. The invention in general relates to the use of an oligonucleotide according to the invention for the treatment or the prevention of diseases, in which VEGF is overexpressed. Such diseases in which VEGF is over expressed are for example cancer, age-related macular degeneration, diabetic retinopathy, psoriasis, rheumatoide arthritis and other inflammatory diseases.

The invention furthermore relates to the use of the oligonucleotide as pharmaceutical and to the use of the oligonucleotide for preparing a pharmaceutical composition. In particular, the oligonucleotide can be used in a pharmaceutical composition, which is employed for preventing and/or treating diseases which are associated with the expression or an overexpression (increased expression) of VEGF and for treating of diseases in which VEGF or its overexpression is the causative factor or is involved.

The invention furthermore relates to a pharmaceutical composition which comprise an oligonucleotide and/or its physiologically tolerated salts in addition to pharmaceutically unobjectable excipients or auxiliary substances.

The invention relates to a pharmaceutical composition which comprises at least one oligonucleotide according to the invention that can be used for the treatment of diseases which are associated with abnormal vascular permeability, cell proliferation, cell permeation, angiogenesis, neovascularization, tumor cell growth and the metastasis of neoplastic cells.

The invention further relates to a method for preparing a pharmaceutical composition, which comprises mixing of one or more oligonucleotides according to the invention with physiologically acceptable exipient and optionally additional substances, e.g. if appropiate with suitable additives and/or auxiliaries.

The invention relates in particular to the use of an oligonucleotide or a pharmaceutical composition prepared thereof for the treatment of cancer, e.g. for inhibiting tumor growth and tumor metastasis, and for the treatment of diabetic retinopathy, age-related macular degeneration, psoriasis, rheumatoid arthritis and other inflammatory diseases. For example the oligonucleotide or a pharmaceutical composition prepared thereof may be used for the treatment of solid tumors, like breast cancer, lung cancer, head and neck cancer, brain cancer, abdominal cancer, colon cancer, colorectal cancer, Esophagus cancer, gastrointestinal cancer, Glioma, liver cancer, tongue cancer, neuroblastoma, osteosarcoma, ovarian cancer, pancreatic cancer, prostata cancer, Retinoblastoma, Wilm's tumor, multiple myeloma and for the treatment of skin cancer, like melanoma, for the treatment of lymphomas and blood cancer. The invention further relates to the use of an oligonucleotide according to the invention or a pharmaceutical composition prepared thereof for inhibiting VEGF expression and/or for inhibiting accumulation of ascites fluid and pleural effusion in different types of cancer e.g. breast cancer, lung cancer, head cancer, neck cancer, brain cancer, abdominal cancer, colon cancer, colorectal cancer, Esophagus cancer, gastrointestinal cancer, Glioma, liver cancer, tongue cancer, neuroblastoma, osteosarcoma, ovarian cancer, pancreatic cancer, prostata cancer, Retinoblastoma, Wilm's tumor, multiple myeloma, skin cancer, melanoma, lymphomas and blood cancer. Due to the inhibitory effect on VEGF expression and/or ascites fluid and pleural effusion, an oligonucleotide according to the invention or a pharmaceutical composition prepared thereof can enhance the quality of live. In a preferred embodiment of the invention, the oligonucleotide or a pharmaceutical composition thereof can inhibits accumulation of ascites fluids in ovarian cancer.

The invention furthermore relates to the use of an oligonucleotide or a pharmaceutical composition thereof, e.g. for treating cancer or for preventing tumor metastasis, or for treating age-related macular degeneration, rheumatoid arthritis, psoriasis and diabetic retinopathy in combination with other pharmaceuticals and/or other therapeutic methods, e.g. with known pharmaceuticals and/or known therapeutic methods, such as for example those, which are currently employed for treating cancer and/or for preventing tumor metastasis. Preference is given to a combination with radiation therapy and chemotherapeutic agents, such as cis-platin, cyclophosphamide, 5-fluorouracil, adriamycin, daunorubicin or tamoxifen.

The oligonucleotide and/or its physiologically tolerated salt can be administered to an animal, preferably a mammalian, and in particular a human, on its own, in mixture with another oligonucleotide (or its physiologically tolerated salt), or in the form of a pharmaceutical composition which permit topical, percutaneous, parenteral or enteral use and which comprise, as the active constituent, an effective dose of at least one oligonucleotide in addition to customary pharmaceutically unobjectable excipients and auxiliary substances. Such pharmaceutical composition normally comprises from about 0.1 to 90% by weight of the therapeutically active oligonucleotide(s). The dose can vary within wide limits and is to be adjusted to the individual circumstances in each individual case. In order to treat psoriasis, preference is given to a topical use. In the case of cancer, preference is given to infusions, oral and rectal administration, or nasal application in an aerosol, preferable in the case of lung cancer, while in the case of diabetic retinopathy, preference is given to a topical, intravitreal and oral administration.

A pharmaceutical composition might be prepared in a manner known per se (e.g. Remingtons Pharmaceutical Sciences, Mack Publ. Co., Easton, PA (1985)), with pharmaceutically inert inorganic and/or organic excipients being used. Lactose, corn starch and/or derivatives thereof, talc, stearic acid and/or its salts, etc. can, for example, be used for preparing pills, tablets, coated tablets and hard gelatin capsules. Examples of excipients for soft gelatin capsules and/or suppositories are fats, waxes, semisolid and liquid polyols, natural and/or hardened oils, etc. Examples of suitable excipients for preparing solutions and/or syrups are water, sucrose, invert sugar, glucose, polyols, etc. Suitable excipients for preparing injection solutions are water, alcohols, glycerol, polyols, vegetable oils, etc. Suitable excipients for microcapsules, implants and/or rods are mixed polymers of glycolic acid and lactic acid. In addition, liposome formulations which are e.g. described in N. Weiner, (Drug Develop Ind Pharm 15 (1989) 1523), "Liposome Dermatics^{"} (Springer Verlag 1992) and Hayashi (Gene Therapy 3 (1996) 878). The pharmaceutical composition may also comprise formulation, which enhances the oral availability of the oligonucleotide, such as enhancers of intestinal permeabilization, e.g. mannitol, urea, bile salts, such as CDCA (chenodexoycholate) (2 %).
Dermal administration can also be effected, for example, using ionophoretic methods and/or by means of electroporation. Furthermore, use can be made of lipofectins and other carrier systems, for example those which are used in gene therapy. Systems which can be used to introduce oligonucleotides in a highly efficient manner into eukaryotic cells or into the nuclei of eukaryotic cells are particularly suitable. A pharmaceutical composition may also comprise two or more different oligonucleotides and/or their physiologically tolerated salts and, furthermore, in addition to at least one oligonucleotide, one or more different therapeutically active ingredients.

In addition to the active ingredients and excipients, a pharmaceutical composition can also comprise additives, such as fillers, extenders, disintegrants, binders, lubricants, wetting agents, stabilizing agents, emulsifiers, preservatives, sweeteners, dyes, flavorings or aromatizing agents, thickeners, diluents or buffering substances, and, in addition, solvents and/or solubilizing agents and/or agents for achieving a slow release effect, and also salts for altering the osmotic pressure, coating agents and/or antioxidants.

The dose can vary within wide limits and is to be adjusted to the individual circumstances in each individual case. The pharmaceutical composition may also comprise formulation, which enhances the oral availability of the oligonucleotide, such as enhancers of intestinal permeabilization, e.g. mannitol, urea, bile salts such as CDCA (2 %).

The invention relates to a pharmaceutical composition which comprises at least one oligonucleotide according to the invention that can be used for the treatment of diseases which are associated with abnormal cell proliferation, cell permeation, vascular permeability, angiogenesis, tumor cell growth and the metastasis of neoplastic cells. Such pharmaceutical composition can be used for the treatment and prevention of cancer and metastasis of cancer, the treatment and prevention of psoriasis, and the treatment of diabetic retinopathy.

### Description of the Figures:

Figure 1: Quantitation of VEGF mRNA in U87-MG tumor xenografts grown in mice that were treated with ON 1.
   Tumors were implanted by subcutaneously (s.c.) injecting 2 x 10⁶ U87-MG tumor cells into the flank of each mouse on day 0. The daily i.v. treatment with ON 1 was started on day 4. Tumors were collected on day 18. In tumor sections the mRNA expression levels were assayed by in situ hybridization with a ³⁵S VEGF cRNA probe. For VEGF mRNA quantitation the percentage of the area with greater than 111 dpm/mm² of radioactive probe hybridized in a representative section from each tumor was determined. With the exception of two outliers, there was a clear decrease in VEGF expression in tumors from treated animals (relative to control animals).
Figure 2: Summarizes the results of the concentration dependent effect of different oligonucleotides on the VEGF mRNA level in cells treated with oligonucleotides ON1, ON55 (mismatch) and ON56 (scrambled). VEGF mRNA was quantitated using the ABI Prism 7700 Sequence Detector. The concentration dependent effect on VEGF mRNA amount relative to control cells that were not treated with the oligonucleotides is shown as "1/Fold difference (relative to untreated control)" versus "oligonucleotide concentration [µM]" (from left to right: ON1: bar 1 to 6; ON55: bar 7 to 12; ON56: bar 13 to 18; Control: bar 19 (without oligonucleotide) and 20 (with cellfectin only)). The mismatch (ON55) and scrambled controls (ON56) had no significant effect on the VEGF mRNA levels neither at low nor at high oligonucleotide concentrations, while ON1 decreased the VEGF mRNA level (i.e. lead to an increase in the number of cycles in PCR required to reach a threshold for detection of a PCR product). The effect of ON1 on VEGF mRNA level was concentration dependent. n=4 for each data point, error bars represent standard deviation.
Figure 3: Figure 3 shows the in vivo results of ON1 on tumor xenografts.

This figure summarizes the tumor weights (grams) that were determined on day 18 (on day O U87-MG xenografts were implanted) - each point in this figure indicates the tumor weight that was determined for an individual nude mice. Nude mice were daily i.v. treated with ON1 either at a concentration of O mg/kg, of 4 mg/kg or 12 mg/kg body weight.

### Examples:

### Example 1: Oligonucleotide synthesis

Oligonucleotides (ONs) were synthesized by using an Applied Biosystems 394 DNA synthesizer (Perkin Elmer Applied Biosystems, Inc., Foster City, USA) and standard phosphoramidite chemistry (e.g. F. Eckstein, Ed "Oligonucleotides and Analogues A practical Approach", IRL Press, Oxford, 1991) . After coupling, phosphorothioate linkages were introduced by sulfurization using the Beaucage reagent followed by capping with acetic anhydride and *N*-methylimidazole. After cleavage from the solid support and final deprotection by treatment with concentrated ammonia, ON s were purified by polyacrylamide gel electrophoresis. The *2'-O-methyl* modified ON s were prepared by replacing the standard phosphoramidites in the corresponding cycle with 2'-O-methyl ribonucleoside phophoramidites. All ON s were analyzed by negative ion electrospray mass spectroscopy (Fisons Bio-Q) which in all cases confirmed the calculated mass. The C16-modified oligonucleotides were synthesized using hexadecyloxy (cyanoethoxy) N,N-diisopropyl aminophosphane as phosphitylating reagent in the last step of oligonucleotide synthesis in place of a standard amidite, or by starting from a correspondingly derivatized solid support. The tetraethylene glycol linker is commercially available from Glen Research Corporation. The 2'-phosphoramidite of adenosin or cordycepin were obatined from Chem Genes Corporation and Chemogen Corporation, respectively. The introduction of 5'-phosphates or thiophosphate residues was carried out as described previously (Uhlmann and Engels (1956) Tetrahedron Lett. 27, 1023).

Analysis of the oligonucleotides was done by
a) Analytical gel electrophoresis in 20% acrylamide, 8M urea, 45µM tris-borate buffer, pH 7.0 and/or
b) HPLC-analysis: Waters GenPak FAXcolumn, gradient CH₃CN (400ml), H₂O (1.6l), NaH₂PO₄ (3.1g), NaCl (11.7g), pH6.8 (0.1M an NaCl) after CH₃CN (400ml), H₂O (1.6l), NaH₂PO₄ (3.1g), NaCl (175.3g), pH6.8 (1.5M an NaCl) and/or
c) capillary electrophoresis using a Beckmann capillary eCAP™, U100P Gel Column, 65 cm length, 100 mm I.D., window 15 cm from one end, buffer [140 µM Tris, 360mM borate, 7M urea] and/or
d) negative ion electrospray mass spectrometry which in all cases confirmed the expected mass values.

The methods for analysing oligonucleotides according to a), b), c) and d) are known to a person of skill in the art. These methods are for example described in Schweitzer and Engel "Analysis of oligonucleotides" (in "Antisense - from technology to therapy", a laboratory manual and textbook, Schlingensiepen et al. eds., Biol. Science Vol. 6 (1997) p. 78- 103).

The following oligonucleotides were prepared (see description):
- ON 62:: 3'-G*T*A*C*C*TAC*AGA*TAGT*C*GC*GT*C*GAT*GAC*GGT*AGG-5 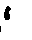
- ON 63:: 3'-C*C*T*AC*AGAT*AGT*C*GC*GT*C*GAT*GAC*G*G-5 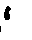
- ON 64:: 3'-C*A*GA*TAGT*C*G*CGT*C*GAT*GAC*G*G-5 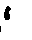
- ON 65:: 3'-A*G*T*CGC*GT*C*GA*T*GAC*G*G-5 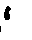
- ON 66:: 3'-C*T*A*CAGA*TAGT*C*GC*GT*C*G-5 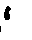
- ON 67:: 3'-G*T*AC*C*TAC*AGAT*AGT*C*GC*GT*C*GAT*GAC*G*G-5 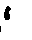
- ON 68:: 3'-G*T*AC*C*TAC*AGAT*AGT*C*GC*G*T-5 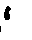
- ON 1:: 3'-G*T*A*C*C*TA*C*AGA*T*AGT*CG*C*G
- ON 69:: 3'-G*T*A*C*C*TA*C*AGA*T*AGT*C*G*C-5 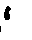
- ON 70:: 3'-A*C*C*T*AC*AGA*T*AGT*C*G*C*G-5 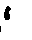
- ON 71:: 3'-G*T*A*C*C*TA*C*AGA*T*AG*T*C*G-5 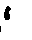
- ON 72:: 3'-T*A*C*C*TAC*AGA*T*AG*T*CG*C*G-5 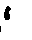
- ON 73:: 3'-T*A*C*C*TAC*AGA*T*AG*T*C*G*C-5 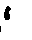
- ON 8:: 5'-G C*G C*T G A*T A G A*C*A T*C*C A*T*G-3'
- ON 9:: 5'-G*C*G C*T G A*T*A G A*C*A T*C*C A*T*G-3'
- ON 10:: 5'-G*C*G C*T G A*T*A G A*C*A*T*C C*A*T*G-3'
- ON 11:: 5'-G*C*G*C*T G A*T*A G A*C*A T*C*C*A*T*G-3'
- ON 12:: 5'-G*C*G*C*T G A*T*A G A*C A*T*C*C*A*T*G-3'
- ON 13:: 5'-G*C*G C*T G A*T*A G A*C*A T*C*C*A*T*G-3'
- ON 14:: 5'-G*C*G*C*T G A*T*A G A*C*A*T*C*C*A*T*G-3'
- ON 15:: 5'-G*C*G*C*T G A*T*A G A*C*A*T*C*C*A*T*G-3'
- ON 2:: 5'-G^{*}C G*C*T*G*A*T*A G*A*C*A*T*C*C*A*T*G-3'
- ON 28:: 5'-G*C*G C*T G A*T*A G A*C*A T*C*C*A*T*G-3'
- ON 29:: 5'-G*C*G C*T G A*T*A G A*C*A T*C*C*A*T*G-3'
- ON 30:: 5'-G*C*G C*T G A*T*A G A*C*A T*C*C*A*T*G-3'
- ON 31:: 5'-G*C*G C*T G A*T*A G A*C*A T*C*C*A*T*G-3'
- ON 32:: 5'-G*C*G C*T G A*T*A G A*C*A T*C*C*A*T*G-3'
- ON 33:: 5'-G*C*G C*T G A*T*A G A*C*A T*C*C*A*T*G-3'
- ON 34:: 5'-G*C*G C*T G A*T*A G A*C*A T*C*C*A*T*G-3'
- ON 35:: 5'-G*C*G C*T G A*T*A G A*C*A T*C*C*A*T*G-3'
- ON 36:: 5'-G*C*G C*T G A*T*A G A*C*A T*C*C*A*T*G-3'
- ON 37:: 5'-G*C*G C*T G A*T*A G A*C*A T*C*C*A*T*G-3'
- ON 48:: 5'-G*C*G C*T G A*T*A G A*C*A T*C*C*A*T*G-C₁₆-3'
- ON 49:: 5'-C₁₆-G*C*G C*T G A*T*A G A*C*A T*C*C*A*T*G-3'
- ON53:: 5'-p*(2'5'-Co Co Co Co)(teg) G*C*G C*T G A*T*A G A*C*A T*C*C*A*T*G -3*'*
- ON54:: 5'-p*-(2'5'-rA*rA*rA*rA)*(teg) G*C*G C*T G A*T*A G A*C*A T*C*C*A*T*G -3*'*
wherein
"teg" is triethyleneglycol (linker),
"N" is 2'-O-methylribonucleoside,
"rA" is ribo-A (2 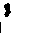 5 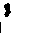 -linked adenylate),
"Co" is 3'-deoxy-A (Cordycepin) (2 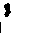 5 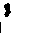 -linked 3 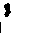 -deoxy adenylate),
"p*" is 5'-thiophosphate,
"*" is phosphorothioate.

### Example 2: Detailed description of the synthesis of ON1

### 5'-G*C*G C*T G A*T*A G A*C*A T*C*C*A*T*G -3' (with "*" phosphorothioate)

The partially phosphorothioated oligonucleotide ON1 with 12 phosphorothioate internucleoside linkages was synthesized on an ABI 390Z DNA synthesizer (Perkin Elmer - Applied Biosystems, Foster City , USA) using a controlled pore glass (CPG) support. The aminopropylated CPG was loaded with 200 µmol of 5'-O-Dimethoxytrityl-N²-isobutyroyl-deoxygunanosine-3'-O-succinate. After removal of the 5'-dimethoxytrityl group with 3% trichloroacetic acid in dichloromethane, the second base (T) was coupled using the corresponding 5'-O-Dimethoxytrityl-thymidine-3'-O-(β-cyanoethoxy)-N,N-diisopropylamino phosphoramidite using a synthesis cycle as provided by the supplier (ABI). For the introduction of the phosphorothioate linkages 3H-, 1.2-benzodi-thiol-3-on1.1-dioxide (0.075M Beaucage's reagent) was used in the cycle instead of the iodine/pyridine/water, which is used for introduction of the phosphodiester linkage. The capping reaction with acetic acid anhydride was performed directly after the coupling reaction in case of the phosphodiesters, but after Beaucage sulfurizatuion in case of the phosphorothioate linkages. After complete chain elongation, the oligonucleotide was cleaved from the CPG and deprotected by treatment with 150 ml concentrated ammonia for 16 hours at 50°C. The crude oligonucleotide (19200 OD₂₆₀) was purified by precipitation from n-butanol and subsequent FPLC on a Q SepharoseR High Performance column (60/100; Pharmacia) using a Pharmacia Biopilot system. The oligonucleotide was eluted with 0.45 - 1.0 M NaCl gradient in 10 mM NaOH at pH 12 within 77 minutes. Oligonucleotide containing fractions were analyzed by HPLC on a Gen-Pak Fax column (Millipore-Waters) using a NaCl-gradient (buffer A: 10mM NaH₂PO₄ 100mM NaCl in acetonitrile/water = 1:4/v:v pH 6.8; buffer B: 10 mM NaH₂PO_{4,} 1.5 M NaCl in acetonitrile/water = 1:4/v:v; 5 to 40 % B in 30 minutes). Homogeneous fractions were combined (5660 OD₂₆₀) and desalted by ultra-filtration. After a second desalting step by precipitation fron ethanol/isoprpanol and lyophilization the oligomer was obtained as a white foam (165 mg). The oligonucleotide was characterized by negative ion electrospray mass spectrometry (calc. 6005.6; measured 6006.1) Furthermore, the oligomer was analyzed by capillary electrophoresis in polyacrylamide gels (U 100P gel capillary from Beckman Instruments; ID 100 µM; buffer: 7M urea, 140 mM tris/borate) by applying the compound (1 OD/ml) at 10 kV for 4 seconds and developing the electropherogram at const. 11 kV for 40 minutes.

### Example 3: Detailed description of the synthesis of ON28

### 5'-G*C*G C*T G A*T*A G A*C*A T*C*C*A*T*G-3' (with "*" phosphorothioate and N = 2'-O-methylribonucleoside T = 2'-O-methyl-U

The partially 2'-O-methyl modified oligonucleotide was synthesized as described in example 2, starting from a CPG support which was loaded with 5'-O-Dimethoxytrityl-N²-isobutyroyl-2'-O-methyl-gunanosine-3'-O-succinate. For the introduction of the 2'-O-methyl ribonucleosides, the corresponding nucleoside-2'-O-methyl-3'-phosphoramidites are coupled instead of the normal deoxynucleoside-3'-phosphoramidites. The crude oligonucleotide (18700 OD₂₆₀) is precipitated from - n-butanol (594 mg). The oligonucleotide was characterized by negative ion electrospray mass spectrometry (calc. 6293.9; measured 6292.9).

### Example 4: Treatment of cells with antisense oligonucleotides

The cells are plated in 96-well plates at 30,000 cells/well, 150 ul medium per well (medium depends on cell type). The next day, Cellfectin (Gibco-BRL) is diluted to 400 ug/ml in water (solution A). Oligonucleotides are diluted to 40X the final desired concentration in water (solution B). Equal amounts of solutions A and B are mixed, to give the desired volume of a solution that is 200 µg/ml Cellfectin and 20X oligonucleotide, and the mixture left at room temperature for 30 minutes. After 30 minutes, 19 volumes of Optimem (Gibco-BRL) is added to give a final solution that is 10 µg/ml Cellfectin and 1X oligonucleotide (solution C). Medium is removed from the cells, the wells are washed 2X with Optimem, and 150 µl solution C added to each well. The plates are then returned to the incubator (37°C, 5% CO₂). After 5 hours, the Cellfectin/oligonucleotide solution is removed and replaced with 150 µl of regular growth medium. VEGF protein and mRNA assays are performed beginning 19 hours later.

### Example 5: VEGF protein assay

Samples (from example 4) of conditioned medium are taken from the desired wells and assayed for the presence of human VEGF using the human VEGF ELISA kit from R & D systems.The assay protocol is the one provided with the kit.

### Example 6: VEGF mRNA assay

From the cells from example 4, medium is removed from the 96 well plates described above, and cell lysates are prepared from the remaining cells for quantitation of VEGF mRNA by the Applied Biosystems 7700 Analyser.

Quantitation of mRNA follows. The mRNA is purified from the cells and cDNA is produced using Promega's PolyATract Series 9600 mRNA Isolation and cDNA Synthesis Systems (Catalog # Z3790). The instructions provided with the kit were followed.
The quantity of VEGF cDNA was measured using the Perkin Elmer / Applied Biosystems ABI Prism 7700 Sequence Detection System. The Perkin Elmer / Applied Biosystems TaqMan ™ PCR reagent kit (Catalog # N808-0230) was used to set up the reactions. The Perkin Elmer / Applied Biosystems TaqMan β-actin Control Reagents kit (Catalog # 401846) was used to set up the B-actin control reactions. The VEGF data was normalized against the β-actin data. The sequences of the fluorescent tagged probe and the primers designed for the VEGF reactions are:
SEQ ID NO. 20:
   Probe: 5*'*- 6FAM-TCAGCGCAGCTACTGCCATCCAAT-TAMRA -3*'* (5 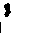 →3 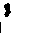 )
SEQ ID NO. 21:
   Forward Primer 1: 5*'*- GGA GGG CAG AAT CAT CAC GAA -3*'* (5 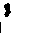 →3 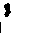 )
SEQ ID NO. 22:
   Reverse Primer 2: 5*'*- AGG GTA CTC CTG GAA GAT GTC CAC-3*'* (5 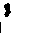 →3 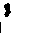 )

### Example 7: Determination of IC(50)- values

The IC50s are calculated based on a value of 100% for the amount of VEGF protein or mRNA in cells treated with Cellfectin but no oligonucleotide.

### Example 8: In vivo studies

Experiments are performed with 4 - 6 week old female nude (nu/nu) mice. Tumors are grown by subcutaneous implantation of cells (2,000,000 cells in 200 µl for U87-MG). Oligonucleotides are dissolved in phosphate buffered saline and injected subcutaneously or intravenously (tailvein) in a volume of 100 µl. 2 x 10⁶ U87-MG. Some oligonucleotides were also tested by oral administration.

The tumor cells are implanted s.c. on day 0. The drug treatment was administered by daily i.v. tailvein injection. Each treatment group contained 6-10 animals.

When ON 1 was used for the treatment of mice, tumor growth in mice was inhibited/reduced at a concentration of 4 to 12 mg oligonucleotide per kg body weight. ON 1 significantly inhibits the growth of U87-MG tumor xenografts grown subcutaneously in nude mice when administered daily by i.v. or s.c. injection in a dose dependant manner. This is clearly demonstrated by the results shown in table 2.

At the end of the study, the tumors were parrafin-embedded, sectioned, and evaluated for VEGF mRNA expression by in situ hybridization. The amount of VEGF mRNA expression varies within a tumor, with some regions showing very high expression, and others showing no detectable expression. Therefore, VEGF expression in the tumor sections was analysed by quantitating the percentage of the area with a high level of expression. Treatment with ON 1 led to a dramatic decrease in VEGF mRNA levels in tumors when analysed by this method (Figure 1). Microvessel density in the tumors from this study was evaluated by factor VIII staining. There was only a slight decrease in the number of vessels/area in tumors from the treated animals. However, there was a large decrease in the size of the vessels in tumors from the drug-treated animals.

**Table 1**

| **Oligonucleotide** | **Type** | **IC50 for VEGF secretion** | **IC**_{**50**} **for VEGF mRNA** | **IC**_{**50**} **for B-actin mRNA** |
|---|---|---|---|---|
| ON 1 | Partial PS | 300 nM | 100 nM | No effect on mRNA level |
| ON 28 | 2'-O-methyl ribonucleoside gapmer, partial PS | 300 nM | | |
| ON 29 | 2'-O-methyl ribonucleoside chimera, partial PS | 500 nM | | |
| ON 53 | 2'5(Co)₄-conjugate all-2'-O-methyl | 1500 nM | | |
| ON 54 | 2'5'(rA)₄-conjugate all-2'-O-methyl | 230 nM | | |
| ON 55 | 4X mismatch with respect to ON1 control, partial PS | > 3 µM | No effect on mRNA level | No effect on mRNA level |
| ON 56 | Scrambled sequence control, (with respect to ON1) partial PS | > 3 µM | No effect on mRNA level | No effect on mRNA level |
| PS: Phosphorothioate internucleoside bridge | | | | |

**Table 2**

| Reduction of tumor volume during treatment with ON 1. | | | | | | |
|---|---|---|---|---|---|---|
| Tumors were implanted by s.c. injection of 2 x 10⁶ U87-MG cells into a mice on day 0. Then drug treatment was started on day 4. The drug was administered by daily i.v. tailvein injection. Each treatment group contained 6-10 mice. The Data are presented as mean +/- SE (standard error). | | | | | | |

| | daily i.v. injection | | | | | |
|---|---|---|---|---|---|---|
| DAY | 0 mg/kg volume | ± SE | 4mg/kg volume | ± SE | 12mg/kg volume | ± SE |
| 0 | 0 | 0 | 0 | 0 | 0 | 0 |
| 6 | 31 | 3 | 30 | 3 | 26 | 3 |
| 11 | 123 | 16 | 69 | 14 | 39 | 4 |
| 17 | 868 | 171 | 344 | 70 | 236 | 54 |

- drug treatment started at day 4 in all cases
- drug administered by i.v. tailvein injection
- tumor model: 2,000,000 U87-MG cells implanted subcutaneously on day 0
- tumor volume was determined on day 17.

Table 3: Nucleotide sequence of human VEGF (SEQ ID NO. 19)

CAGTGTGCTGGCGGCCCGGCGCGAGCCGGCCCGGCCCCGGTCGGGCCTCCGAAACC
ATGAACTTTCTGCTGTCTTGGGTGCATTGGAGCCTCGCCTTGCTGCTCTACCTCCA
CCATGCCAAGTGGTCCCAGGCTGCACCCATGGCAGAAGGAGGAGGGCAGAATCATC
ACGAAGTGGTGAAGTTCATGGATGTCTATCAGCGCAGCTACTGCCATCCAATCGAG
ACCCTGGTGGACATCTTCCAGGAGTACCCTGATGAGATCGAGTACATCTTCAAGCC
ATCCTGTGTGCCCCTGATGCGATGCGGGGGCTGCTGCAATGACGAGGGCCTGGAGT
GTGTGCCCACTGAGGAGTCCAACATCACCATGCAGATTATGCGGATCAAACCTCAC
CAAGGCCAGCACATAGGAGAGATGAGCTTCCTACAGCACAACAAATGTGAATGCAG
ACCAAAGAAAGATAGAGCAAGACAAGAAAATC

(1) GENERAL INFORMATION:

(i) APPLICANT:
(A) NAME: Hoechst Marion Roussel Deutschland GmbH
(B) STREET: -
(C) CITY: Frankfurt
(D) STATE: -
(E) COUNTRY: Germany
(F) POSTAL CODE (ZIP): 65926
(G) TELEPHONE: 069-305-7072
(H) TELEFAX: 069-35-7175
(I) TELEX: -

(ii) TITLE OF INVENTION: Antisense Oligonucleotides for the Inhibition
of VEGF Expression

(iii) NUMBER OF SEQUENCES: 22

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 1:

GCGCTGATAG ACATCCATG 19

(2) INFORMATION FOR SEQ ID NO: 2:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..33

(xi) SEQUENCE DESCRIPTION: SEQ ID NO: 2:

CATGGATGTC TATCAGCGCA GCTACTGCCA TCC 33

(2) INFORMATION FOR SEQ ID NO: 3:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 3:

CATGGATGTC TATCAGCGC 19

(2) INFORMATION FOR SEQ ID NO: 4:

(i) SEQUENCE CHARACTERISTICS:
(A) LENGTH: 33 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..33

(xi) SEQUENCE DESCRIPTION: SEQ ID NO: 4:

GTACCTACAG ATAGTCGCGT CGATGACGGT AGG 33

(2) INFORMATION FOR SEQ ID NO: 5:

(i) SEQUENCE CHARACTERISTICS:
(A) LENGTH: 26 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..26

(xi) SEQUENCE DESCRIPTION: SEQ ID NO: 5:

CCTACAGATA GTCGCGTCGA TGACGG 26

(2) INFORMATION FOR SEQ ID NO: 6:

(i) SEQUENCE CHARACTERISTICS:
(A) LENGTH: 22 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..22

(xi) SEQUENCE DESCRIPTION: SEQ ID NO: 6:

CAGATAGTCG CGTCGATGAC GG 22

(2) INFORMATION FOR SEQ ID NO: 7:

(i) SEQUENCE CHARACTERISTICS:
(A) LENGTH: 17 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..17

(xi) SEQUENCE DESCRIPTION: SEQ ID NO: 7:

AGTCGCGTCG ATGACGG 17

(2) INFORMATION FOR SEQ ID NO: 8:

(i) SEQUENCE CHARACTERISTICS:
(A) LENGTH: 18 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 8:

CTACAGATAG TCGCGTCG 18

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..29

(xi) SEQUENCE DESCRIPTION: SEQ ID NO: 9:

GTACCTACAG ATAGTCGCGT CGATGACGG 29

(2) INFORMATION FOR SEQ ID NO: 10:

(i) SEQUENCE CHARACTERISTICS:
(A) LENGTH: 20 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..20

(xi) SEQUENCE DESCRIPTION: SEQ ID NO: 10:

GTACCTACAG ATAGTCGCGT 20

(2) INFORMATION FOR SEQ ID NO: 11:

(i) SEQUENCE CHARACTERISTICS:
(A) LENGTH: 19 base pairs

(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 11:

GTACCTACAG ATAGTCGCG 19

(2) INFORMATION FOR SEQ ID NO: 12:

(i) SEQUENCE CHARACTERISTICS:
(A) LENGTH: 18 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 12:

GTACCTACAG ATAGTCGC 18

(2) INFORMATION FOR SEQ ID NO: 13:

(i) SEQUENCE CHARACTERISTICS:
(A) LENGTH: 17 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..17

(xi) SEQUENCE DESCRIPTION: SEQ ID NO: 13:

ACCTACAGAT AGTCGCG 17

(2) INFORMATION FOR SEQ ID NO: 14:

(i) SEQUENCE CHARACTERISTICS:
(A) LENGTH: 17 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..17

(xi) SEQUENCE DESCRIPTION: SEQ ID NO: 14:

GTACCTACAG ATAGTCG 17

(2) INFORMATION FOR SEQ ID NO: 15:

(i) SEQUENCE CHARACTERISTICS:
(A) LENGTH: 18 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..18

(xi) SEQUENCE DESCRIPTION: SEQ ID NO: 15:

TACCTACAGA TAGTCGCG 18

(2) INFORMATION FOR SEQ ID NO: 16:

(i) SEQUENCE CHARACTERISTICS:
(A) LENGTH: 17 base pairs
(B) TYPE: nucleic acid
(C) STRANDEDNESS: single
(D) TOPOLOGY: linear
(E) This sequence is written in 3′→5′ direction

(ii) MOLECULE TYPE: DNA (genomic)

(ix) FEATURE:
(A) NAME/KEY: exon
(B) LOCATION:1..17

(xi) SEQUENCE DESCRIPTION: SEQ ID NO: 16:

TACCTACAGA TAGTCGC                                                    17

(2) INFORMATION FOR SEQ ID NO: 17:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 17:

GCGACGATAG ATCTCCATG                                                   19

(2) INFORMATION FOR SEQ ID NO: 18:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..19

(xi) SEQUENCE DESCRIPTION: SEQ ID NO: 18:

CGAAGCACTG TACGCATTG                                                   19

(2) INFORMATION FOR SEQ ID NO: 19:

(i) SEQUENCE CHARACTERISTICS:
(A) LENGTH: 480 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..480

(xi) SEQUENCE DESCRIPTION: SEQ ID NO: 19:

CAGTGTGCTG GCGGCCCGGC GCGAGCCGGC CCGGCCCCGG TCGGGCCTCC GAAACCATGA 60

ACTTTCTGCT GTCTTGGGTG CATTGGAGCC TCGCCTTGCT GCTCTACCTC CACCATGCCA 120

AGTGGTCCCA GGCTGCACCC ATGGCAGAAG GAGGAGGGCA GAATCATCAC GAAGTGGTGA 180

AGTTCATGGA TGTCTATCAG CGCAGCTACT GCCATCCAAT CGAGACCCTG GTGGACATCT 240

TCCAGGAGTA CCCTGATGAG ATCGAGTACA TCTTCAAGCC ATCCTGTGTG CCCCTGATGC 300

GATGCGGGGG CTGCTGCAAT GACGAGGGCC TGGAGTGTGT GCCCACTGAG GAGTCCAACA 360

TCACCATGCA GATTATGCGG ATCAAACCTC ACCAAGGCCA GCACATAGGA GAGATGAGCT 420

TCCTACAGCA CAACAAATGT GAATGCAGAC CAAAGAAAGA TAGAGCAAGA CAAGAAAATC 480

(2) INFORMATION FOR SEQ ID NO: 20:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..24

(xi) SEQUENCE DESCRIPTION: SEQ ID NO: 20:

TCAGCGCAGC TACTGCCATC CAAT 24

(2) INFORMATION FOR SEQ ID NO: 21:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..21

(xi) SEQUENCE DESCRIPTION: SEQ ID NO: 21:

GGAGGGCAGA ATCATCACGA A 21

(2) INFORMATION FOR SEQ ID NO: 22:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..24

(xi) SEQUENCE DESCRIPTION: SEQ ID NO: 22:

AGGGTACTCC TGGAAGATGT CCAC 24

## Claims

1. An oligonucleotide or a derivative thereof which has the sequence SEQ ID NO. 4 or a part thereof, wherein
SEQ ID NO. 4 is 3'-GTACCTACAGATAGTCGCGTCGATGACGGTAGG-5 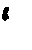 ,
with
the first proviso, that not all internucleoside bridges in the oligonucleotide are phosphodiester internucleoside bridges and not all phosphodiester internucleoside bridges are replaced by phosphorothioate internucleoside bridges and/or
the second proviso, that the oligonucleotide contains no modified nucleosides selected from C5-propynyl uridine, C5-propynyl cytidine, C5-hexynyl uridine, C5-hexynyl cytidine, 6-aza uridine and 6-aza cytidine.

2. An oligonucleotide as claimed in claim 1, which has a length of 17 to 33 nucleotides.

3. An oligonucleotide as claimed in one or more of claims 1 and 2, which has one of the sequences SEQ ID NO. 5 to SEQ ID NO. 16, wherein
SEQ ID NO. 5 is 3'-CCTACAGATAGTCGCGTCGATGACGG-5 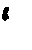 ,
SEQ ID NO. 6 is 3'-CAGATAGTCGCGTCGATGACGG-5 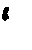 ,
SEQ ID NO. 7 is 3'-AGTCGCGTCGATGACGG-5 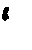 ,
SEQ ID NO. 8 is 3'-CTACAGATAGTCGCGTCG-5 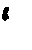 ,
SEQ ID NO. 9 is 3'-GTACCTACAGATAGTCGCGTCGATGACGG-5 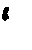 ,
SEQ ID NO. 10 is 3'-GTACCTACAGATAGTCGCGT-5 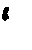 ,
SEQ ID NO. 11 is 3'-GTACCTACAGATAGTCGCG-5',
SEQ ID NO. 12 is 3'-GTACCTACAGATAGTCGC-5 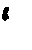 ,
SEQ ID NO. 13 is 3'-ACCTACAGATAGTCGCG-5 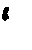 ,
SEQ ID NO. 14 is 3'-GTACCTACAGATAGTCG-5 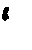 ,
SEQ ID NO. 15 is 3'-TACCTACAGATAGTCGCG-5 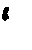 and
SEQ ID NO. 16 is 3'-TACCTACAGATAGTCGC-5 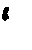 .

4. An oligonucleotide as claimed in one or more of claims 1 to 3, wherein the oligonucleotide has one or more modifications and wherein each modification is located at a particular phosphodiester internucleoside bridge and/or of a particular β-D-2*'*-deoxyribose unit and/or a particular natural nucleoside base position in comparison to an oligonucleotide of the same sequence which is composed of natural DNA.

5. An oligonucleotide as claimed in one or more of claims 1 to 4, wherein the terminal 1 to 5 nucleotide units at the 5*'* end and/or at the 3*'* end of the oligonucleotide are protected by modifying internucleoside bridges located at the 5*'*and/or the 3*'* end of the corresponding nucleosides.

6. An oligonucleotide as claimed in one or more of claims 1 to 5, wherein at least one internal pyrimidine nucleoside and/or an internucleoside bridge located at the 5*'*end and/or the 3*'*end of this pyrimidine nucleoside is modified.

7. An oligonucleotide as claimed in one or more of claims 1 to 6, wherein each modification is independently selected from
a) the replacement of a phosphodiester internucleoside bridge located at the 3*'*-and/or the 5*'*- end of a nucleoside by a modified internucleoside bridge,
b) the replacement of phosphodiester bridge located at the 3*'*- and/or the 5*'*-end of a nucleoside by a dephospho bridge,
c) the replacement of a sugar phosphate unit from the sugar phosphate backbone by another unit,
d) the replacement of a β-D-2*'*-deoxyribose unit by a modified sugar unit,
e) the replacement of a natural nucleoside base by a modified nucleoside base,
f) the conjugation to a molecule which influences the properties of the oligonucleotide, g) the conjugation to a 2 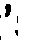 5 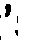 -linked oligoadenylate molecule or a derivative thereof, optionally via an appropriate linker molecule, and
h) the introduction of a 3*'*-3*'* and/or a 5*'*-5*'* inversion at the 3*'* and/or the 5*'* end of the oligonucleotide.

8. An oligonucleotide as claimed in one or more of claims 1 to 7, wherein each modification is independently selected from
a) the replacement of a phosphodiester internucleoside bridge located at the 3*'*-and/or the 5*'*- end of a nucleoside by a modified internucleoside bridge, wherein the modified internucleoside bridge is selected from phosphorothioate, phosphorodithioate, NR¹R¹ 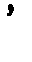 -phosphoramidate, boranophosphate, phosphate-(C₁-C₂₁)-O-alkyl ester, phosphate-[(C₆-C₁₂)aryl-((C₁-C₂₁) -O-alkyl]ester, (C₇-C₁₂)-α-hydroxmethylaryl, (C₁-C₈)alkylphosphonate and/or (C₆-C₁₂)-arylphosphonate bridges,
wherein R¹ and R¹ 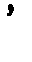 are, independently of each other, hydrogen, (C₁-C₁₈)-alkyl, (C₆-C₂₀)-aryl, (C₆-C₁₄)-aryl-(C₁-C₈)-alkyl, or
R¹ and R¹ 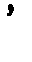 form, together with the nitrogen atom carrying them, a 5-6-membered heterocyclic ring which can additionally contain a further heteroatom from the group O, S and N;
b) the replacement of phosphodiester bridge located at the 3*'*- and/or the 5*'*-end of a nucleoside by a dephospho bridge, wherein the dephospho bridge is selected from formacetal, 3 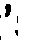 -thioformacetal, methylhydroxylamine, oxime, methylenedimethyl-hydrazo, dimethylenesulfone and silyl groups;
c) the replacement of a sugar phosphate unit from the sugar phosphate backbone by another unit, wherein the other unit is selected from morpholino-derivative units, polyamide nucleic acid backbone units, and phosphomonoacidic ester nucleic acid backbone units; d) the replacement of a β-D-2*'*-deoxyribose unit by a modified sugar unit, wherein the modified sugar unit is selected from β-D-ribose, α-D-2 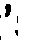 -deoxyribose, L-2 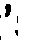 -deoxyribose, 2 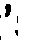 -F-2 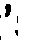 -deoxyribose, 2 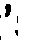 -O-(C₁-C₆)alkyl-ribose, 2 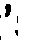 -O-(C₂-C₆)alkenyl-ribose, 2 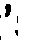 -[O-(C₁-C₆)alkyl-O-(C₁-C₆)alkyl]-ribose, 2 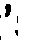 -NH₂-2 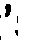 -deoxyribose, β-D-xylo-furanose, α-arabinofuranose, 2,4-dideoxy-β-D-erythro-hexo-pyranose, carbocyclic sugar analogs, open-chain sugar analogs and bicyclosugars;
e) the replacement of a natural nucleoside base by a modified nucleoside base, wherein the modified nucleoside base is selected from 5-(hydroxymethyl)uracil, 5-aminouracil, pseudouracil, dihydrouracil, 5-fluorouracil, 5-fluorocytosine, 5-chlorouracil, 5-chlorocytosine, 5-bromouracil, 5-bromocytosine, 2,4-diaminopurine or 7-deaza-7-substituted and 7-deaza-8-substituted purines and 8-aza purines;
f) the conjugation to a molecule which influences the properties of the oligonucleotide, wherein the molecule which influences the properties of the oligonucleotide is selected from polylysine, intercalating agents, fluorescent agents, crosslinking agents, lipophilic molecules, lipids, steroids, vitamins, poly- or oligo-ethylene glycol, (C₁₂-C₁₈)-alkyl phosphate diesters, O-CH₂-CH(OH)-O-(C₁₂-C₁₈)-alkyl groups;
g) the conjugation to a 2 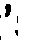 5 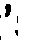 -linked oligoadenylate molecule or a derivative thereof, optionally via an appropriate linker molecule, wherein the 2 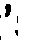 5 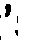 -linked oligoadenylate molecule is selected from triadenylat, tetraadenylate, pentaadenylate, hexaadenyltat and heptaadenylat molecules and derivatives thereof; and
h) the introduction of a 3*'*-3*'* and/or a 5*'*-5*'* inversion at the 3*'* and/or the 5*'* end of the oligonucleotide.

9. An oligonucleotide as claimed in one or more of claims 1 to 8, wherein the oligonucleotide has the sequence SEQ ID NO. 11 and one of the following patterns of internucleoside bridge modifications
ON 1: 3'-G*T*A*C*C*T A*C*A G A*T*A G T*C G*C*G -5',
ON 2: 5'- G*C G*C*T*G*A*T*A G*A*C*A*T*C*C*A*T*G -3*'*,
ON 3: 5'- G*C*G C*T*G A*T*A G A*C*A T*C*C*A*T*G -3*'*,
ON 4: 5'- G*C*G*C*T*G A*T*A G A*C*A T*C*C*A*T*G -3*'*,
ON 5: 5'- G*C G C*T*G A*T*A G A*C*A T*C*C*A*T*G -3*'*,
ON 6: 5'- G*C G C*T G A*T*A G A*C*A T*C*C*A*T*G -3*'*,
ON 7: 5'- G*C^{*}G C*T G A*T*A G A*C*A T*C*C*A*T*G -3*'*,
ON 8: 5'- G C*G C*T G A*T A G A*C*A T*C*C A*T*G -3*'*,
ON 9: 5'- G*C*G C*T G A*T*A G A*C*A T*C*C A*T*G -3*'*,
ON 10: 5'- G*C*G C*T G A*T*A G A*C*A*T*C C*A*T*G -3*'*,
ON 11: 5'- G*C*G*C*T G A*T*A G A*C*A T*C*C*A*T*G -3*'*,
ON 12: 5'- G*C*G*C*T G A*T*A G A*C A*T*C*C*A*T*G -3*'*,
ON 13: 5'- G*C*G C*T G A*T*A G A*C*A T*C*C*A*T*G -3*'*,
ON 14: 5'- G*C*G*C*T G A*T*A G A*C*A*T*C*C*A*T*G -3*'* and
ON 15: 5'- G*C G*C*T*G*A*T*A*G*A*C*A*T*C*C*A*T*G -3*'*,
wherein
"*" indicates the position of a modified internucleoside bridge.

10. An oligonucleotide as claimed in one or more of claims 1 to 9, wherein the oligonucleotide has the sequence SEQ ID NO. 11 and one of the following patterns of nucleoside modifications
ON 16: 5'- G C G C T G A T A G A CA T C C A T G -3',
ON 17: 5'- G C G C T G A T A G A CA T C C A T G -3',
ON 18: 5'- G C G C T G A T A G A CA T C C A T G -3',
ON 19: 5'- G C G C T G A T A G A CA T C C A T G -3',
ON 20: 5'- G C G C T G A T A G A CA T C C A T G -3',
ON 21: 5'-G C G C T G A T A G A CA T C C A T G -3',
ON 22: 5'- G C G C T G A T A G A CA T C C A T G -3',
ON 23: 5'- G C G C T G A T A G A CA T C C A T G -3',
ON 24: 5'- G C G C T G A T A G A CA T C C A T G -3',
ON 25: 5'- G C G C T G A T A G A CA T C C A T G -3',
ON 26: 5'- G C G C T G A T A G A CA T C C A T G -3', and
ON 27: 5'- G C G C T G A T A G A CA T C C A T G -3',
wherein
" N " indicates the position of a modified nucleoside.

11. An oligonucleotide as claimed in one or more of claims 1 to 10, wherein one or more phosphodiester internucleoside bridges are replaced by phosphorothioate bridges and wherein " * " indicates the position of a phosphorothioate internucleoside bridge.

12. An oligonucleotide as claimed in one or more of claims 1 to 11, wherein one or more β-D-2*'*-deoxyribose units are replaced by 2*'*-O-methylribose and wherein " N " indicates the position of a 2*'*-O-methylribo-nucleoside (in this case "T" is 2*'*-O-methyluridine).

13. An oligonucleotide as claimed in one or more of claims 9 to 11, wherein a C16-alkyl group is linked to its 5*'*and/or its 3*'*end.

14. A method of making an oligonucleotide as claimed in one or more of claims 1 to 13 by condensing suitably protected monomers on a solid support.

15. The use of an oligonucleotide as claimed in one or more of claims 1 to 13 for inhibiting the expression of VEGF.

16. A method of inhibiting the expression of VEGF, wherein an oligonucleotide as claimed in one or more of claims 1 to 13 is brought into contact with a VEGF encoding nucleic acid.

17. The use of an oligonucleotide as claimed in one or more of claims 1 to 13 for preparing a pharmaceutical composition.

18. A method of making a pharmaceutical composition by mixing one or more oligonucleotides as claimed in one or more of claims 1 to 13 with a physiologically acceptable exipient and optionally additional substances.

19. A pharmaceutical composition which comprises at least one oligonucleotide as claimed in one or more of claims 1 to 13.

20. The use of a pharmaceutical composition which comprises at least one oligonucleotide as claimed in claims 1 to 13 for the treatment of diseases, which are associated with abnormal vascular permeability, cell proliferation, cell permeation, angiogenesis, neovascularization, tumor cell growth and/or metastasis.

21. The use of a pharmaceutical composition which comprises at least one oligonucleotide as claimed in one or more of claims 1 to 13, in combination with other pharmaceuticals and/or other therapeutic methods.
